# EUROPEAN PATENT SPECIFICATION

(11) **EP 1 682 113 B1**
(45) Date of publication and mention of the grant of the patent: **23.01.2008**
(21) Application number: 04798512.2
(22) Date of filing: 12.11.2004
(51) Int. Cl.: A61K 31/18, A61K 49/00, A61K 51/00

(54) **INHIBITOR IMAGING AGENTS**
HEMMER-DARSTELLENDE MITTEL
AGENTS D'IMAGERIE INHIBITEURS

(30) Priority: 14.11.2003 GB 0326546
(43) Date of publication of application: 26.07.2006
(73) Proprietor: GE Healthcare Limited, Little Chalfont Buckinghamshire HP7 9NA (GB)
(72) Inventor: CUTHBERTSON, Alan, Amersham Health AS, N-0401 Oslo (NO); SOLBAKKEN, Magne, Amersham Health AS, N-0401 Oslo (NO); BJURGERT, Emma, Amersham Health AS, N-0401 Oslo (NO)
(74) Representative: Canning, Lewis R.
(86) International application number: PCT/GB2004/004792
(87) International publication number: WO 2005/049005

(56) References cited:
- EP-A- 0 895 988
- WO-A-20/04069365
- US-A1- 2002 037 916
- US-A1- 2002 090 654

## Description

### Field of the Invention.

The present invention relates to diagnostic imaging agents for *in vivo* imaging. The imaging agents comprise a metalloproteinase inhibitor labelled with an imaging moiety suitable for diagnostic imaging *in vivo.*

### Background to the Invention.

The matrix metalloproteinases (MMPs) are a family of at least 20 zinc-dependent endopeptidases which mediate degradation, or remodelling of the extracellular matrix (ECM) [Massova et al FASEB J 12 1075 (1998)]. Together, the members of the MMP family can degrade all of the components of the blood vessel wall and therefore play a major role in both physiological and pathological events that involve the degradation of components of the ECM. Since the MMPs can interfere with the cell-matrix interactions that control cell behaviour, their activity affects processes as diverse as cellular differentiation, migration, proliferation and apoptosis. The negative regulatory controls that finely regulate MMP activity in physiological situations do not always function as they should. Inappropriate expression of MMP activity is thought to constitute part of the pathological mechanism in several disease states. MMPs are therefore targets for therapeutic metalloproteinase inhibitors (MMPi's) in many inflammatory, malignant and degenerative diseases [Whittaker et al Chem. Rev. 99, 2735 (1999)].

Consequently, it is believed that synthetic inhibitors of MMPs may be useful in the treatment of many inflammatory, malignant and degenerative diseases. Furthermore, it has been suggested that inhibitors of MMPs may be useful in the diagnosis of these diseases. WO 01/60416 discloses chelator conjugates of matrix metalloproteinase (MMP) inhibitors, and their use in the preparation of metal complexes with diagnostic metals. The specific classes of MMP inhibitor described are hydroxamates, especially succinyl hydroxamates. The compounds are proposed to be useful in the diagnosis of cardiovascular pathologies associated with extracellular matrix degradation such as atherosclerosis, heart failure and restenosis. Preferred MMP inhibitors, chelators and linkers are described therein. A report by Zheng et al [NucL Med- BioL 29 761-770 (2002)] documented the synthesis of MMP inhibitors labelled with the positron emission tomography (PET) tracers ¹¹C and ¹⁸F. The compounds described therein are postulated to be useful in the non-invasive imaging of breast cancer.

### The Present Invention.

It has now been found that a particular class of sulphonamide hydroxamate matrix metalloproteinase inhibitors (MMPi's) class labelled with an imaging moiety are useful diagnostic imaging agents for *in vivo* imaging and diagnosis of the mammalian body. These compounds present superior MMP inhibitory activity with Ki in the subnanomolar range. The urinary excretion profiles of the MMPi's of the invention can be adjusted by use of appropriate linker groups, especially polyethyleneglycol (PEG) linker groups.

The imaging agents of the present invention are useful for the *in vivo* diagnostic imaging of a range of disease states (inflammatory, malignant and degenerative diseases) where specific matrix metalloproteinases are known to be involved. These include:
(a) atherosclerosis, where various MMPs are overexpressed. Elevated levels of MMP-1, 3, 7, 9, 11, 12, 13 and MT1-MMP have been detected in human atherosclerotic plaques [S.J. George, Exp. Opin. Invest. Drugs, 9(5), 993-1007 (2000) and references therein] . Expression of MMP-2 [Z. Li et al, Am. J. Pathol., 148, 121-128 (1996)] and MMP-8 [M. P. Herman et al, Circulation, 104, 1899-1904 (2001)] in human atheroma has also been reported;
(b) chronic heart failure (Peterson, J. T. et al. Matrix metalloproteinase inhibitor development for the treatment of heart failure, Drug Dev. Res. (2002), 55(1), 29-44 reports that MMP-1, MMP-2, MMP-3, MMP-8, MMP-9, MMP-13 and MMP-14 are upregulated in heart failure);
(c) cancer [Vihinen et al, Int. J. Cancer 99, p157-166 (2002) reviews MMP involvement in cancers, and particularly highlights MMP-2, MMP-3, MMP-7, and MMP-9];
(d) arthritis [Jacson et al, Inflamm. Res. 50(4), p183-186 (2001) "Selective matrix metalloproteinase inhibition in rheumatoid arthritis-targeting gelatinase A activation", MMP-2 is particularly discussed];
(e) amyotrophic lateral sclerosis [Lim et al, J.Neurochem, 67, 251-259 (1996); where MMP-2 and MMP-9 are involved];
(f) brain metastases, where MMP-2, MMP-9 and MMP-13 have been reported to be implicated [Spinale, Circul.Res., 90 520-530 (2002)];
(g) cerebrovascular diseases, where MMP-2 and MMP-9 have been reported to be involved [Lukes et al, Mol.Neurobiol., 19, 267-284 (1999)];
(h) Alzheimer's disease, where MMP-2 and MMP-9 have been identified in diseased tissue [Backstrom et al, J.Neurochem., 58, 983-992 (1992)];
(i) neuroinflammatory disease, where MMP-2, MMP-3 and MMP-9 are involved *[*Mun-Bryce et al, Brain.Res., 933, 42-49 (2002)];
(j) COPD (ie. chronic obstructive pulmonary disease) where MMP-1, MMP-2, MMP-8 and MMP-9 have been reported to be upregulated [Segura-Valdez et al, Chest, 117, 684-694 (2000)] amongst others;
(k) eye pathology [Kurpakus-Wheater et al, Prog. Histo. Cytochem., 36(3), 179-259 (2001)];
(l) skin diseases [Herouy, Y., Int. J. Mol. Med., 7(1), 3-12 (2001)].

### Detailed Description of the Invention.

In a first aspect, the present invention provides an imaging agent which comprises a metalloproteinase inhibitor of Formula (I) labelled with an imaging moiety, wherein the imaging moiety can be detected following administration of said imaging agent to the mammalian body *in vivo:* 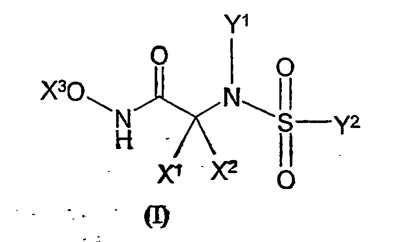 where:
Y¹ is H or -(CH₂)_{w}-(C=O)-Z; where w is an integer of value 1 to 6; and
Z is OH, C₁₋₆ alkoxy, C₄₋₁₀ aryloxy or NR¹R² wherein R¹ and R² are each independently selected from the group consisting ofH, C₁₋₆ alkyl, C₃₋₆ cycloallcyl, C₁₋₆ fluoroalkyl or C₄₋₁₀ aryl.
X¹ and X² together with the carbon atom to which they are attached, form a C₃₋₁₀ saturated ring which may be alicyclic or bicyclic, and may optionally incorporate 1 or 2 heteroatoms chosen from O, N and S;
X³ is H, C₁₋₃ alkyl or C₁₋₃ fluoroalkyl;
Y² is a group of formula -[A¹]ₚ[O]_{q}A² where p and q are 0 or 1, and A¹ is C₁₋₁₀ alkylene, C₃₋₈ cycloalkylene, C₁₋₁₀ perfluoroalkylene, C₆₋₁₀ arylene or C₂₋₁₀ heteroarylene, and A² is H, C₁₋₁₀ alkyl, C₃₋₈ cycloalkyl, C₁₋₁₀ perfluoroalkyl, C₆₋₁₀ aryl or C₂₋₁₀ heteroaryl, with the proviso that when p=0, q is also 0 and A² is not H.

In Formula (I), Y¹ is preferably -(CH₂)_{w}-(C=O)-Z. w is preferably 1, 2 or 3 and is most preferably 2 or 3, ideally 2. X³ is preferably H, CH₃ or CH₂F, and is most preferably H or CH₃, ideally H. Y² is preferably A², where A² is C₆₋₁₀ aryl or C₂₋₁₀ heteroaryl or A¹[O]_{q}A² , where A¹ is C₆₋₁₀ arylene and A² is C₆₋₁₀ aryl or C₂₋₁₀ heteroaryl.

Z is preferably NR¹R², and is most preferably chosen such that one of R¹ and R² is H, and the other is not H.

Suitable monocyclic rings formed by X¹ and X² together with the carbon atom to which they are attached include: cycloalkane (such as cyclopentane or cyclohexane), piperidine, tetrahydrofuran, tetrahydropyran, tetrahydrothiophene and tetrahydropyran. Suitable bicyclic rings include: bicyclo[2.22]octane, bicyclo[2.2.3] nonane and bicyclic tetrahydropyrans having additional ethylene bridges. The rings of X¹ and X² may further optionally comprise one or more hydroxyl, C₁₋₃ alkoxy or C₁₋₃ fluoroalkyl substituents. Preferred rings formed by X¹ and X² together with the carbon atom to which they are attached are C₄₋₆ cycloalkylene, or 4-to 6-membered rings incorporating a single ether linkage, most preferably cyclopentane, cyclohexane or tetrahydropyran rings.

The sulfonamide hydroxamate matrix metalloproteinase inhibitors of the present invention is suitably of molecular weight 100 to 2000 Daltons, preferably of molecular weight 150 to 600 Daltons, and most preferably of molecular weight 200 to 500 Daltons. The inhibitor is preferably of synthetic origin.

The term "labelled with" means that the MMPi itself either comprises the imaging moiety, or the imaging moiety is attached as an additional species, optionally via a linker group, as described for Formula II below. When the MMPi itself comprises the imaging moiety, this means that the 'imaging moiety' forms part of the chemical structure of the MMPi and is a radioactive or non-radioactive isotope present at a level significantly above the natural abundance level of said isotope. Such elevated or enriched levels of isotope are suitably at least 5 times, preferably at least 10 times, most preferably at least 20 times; and ideally either at least 50 times the natural abundance level of the isotope in question, or present at a level where the level of enrichment of the isotope in question is 90 to 100%. Examples of MMPi's comprising the 'imaging moiety' are described below, but include CH₃ groups with elevated levels of ¹³C or ¹¹C and fluoroalkyl groups with elevated levels of ¹⁸F, such that the imaging moiety is the isotopically labelled ¹³C, ¹¹C or ⁸F within the chemical structure of the MMPi. The radioisotopes ³H and ¹⁴C are not suitable imaging moieties.

The "imaging moiety" may be detected either external to the mammalian body or via use of detectors designed for use *in vivo,* such as intravascular radiation or optical detectors such as endoscopes, or radiation detectors designed for intra-operative use. Preferred imaging moieties are those which can be detected externally in a non-invasive manner following administration *in vivo.* Most preferred imaging moieties are radioactive, especially radioactive metal ions, gamma-emitting radioactive halogens and positron-emitting radioactive non-metals, particularly those suitable for maging using SPECT or PET.

The "imaging moiety" is preferably chosen from:
(i) a radioactive metal ion;
(ii) a paramagnetic metal ion,
(iii) a gamma-emitting radioactive halogen;
(iv) a positron-emitting radioactive non-metal;
(v) a hyperpolarised NMR-active nucleus;
(vi) a reporter suitable for *in vivo* optical imaging;
(vii) a β-emitter suitable for intravascular detection.

When the imaging moiety is a radioactive metal ion, ie. a radiometal, The term "radiometal" includes radioactive transition elements plus lanthanides and actinides, and metallic main group elements. The semi-metals arsenic, selenium and tellurium are excluded. Suitable radiometals can be either positron emitters such as ⁶⁴Cu, ⁴⁸V, ⁵²Fe, ⁵⁵CO, ^{94m}Tc or ⁶⁸Ga; γ-emitters such as ^{99m}Tc, ¹¹¹In, ^{113m}In or ⁶⁷Ga. Preferred radiometals are ^{99m}Tc, ⁶⁴Cu, ⁶⁸Ga and ¹¹¹In. Most preferred radiometals are γ-emitters, especially ^{99m}Tc.

When the imaging moiety is a paramagnetic metal ion, suitable such metal ions include: Gd(III), Mn(II), Cu(II), Cr(III), Fe(III), Co(II), Er(II), Ni(II), Eu(III) or Dy(III). Preferred paramagnetic metal ions are Gd(III), Mn(II) and Fe(IIII), with Gd(III) being especially preferred.

When the imaging moiety is a gamma-emitting radioactive halogen, the radiohalogen is suitably chosen from ¹²³I, ¹³¹I or ⁷⁷Br. A preferred gamma-emitting radioactive halogen is ¹²³I.

When the imaging moiety is a positron-emitting radioactive non-metal, suitable such positron emitters include: ¹¹C,¹³N,¹⁵O, ¹⁷F, ¹⁸F, ⁷⁵Br, ⁷⁶Br or ¹²⁴I. Preferred positron-emitting radioactive non-metals are ¹¹C, ¹³N, ¹²⁴I and ¹⁸F, especially ¹¹C and ¹⁸F, most especially ¹⁸F.

When the imaging moiety is a hyperpolarised NMR-active nucleus, such NMR-active nuclei have a non-zero nuclear spin, and include ¹³C, ¹⁵N,¹⁹F, ²⁹Si and ³¹P. Of these, ¹³C is preferred. By the term "hyperpolarised'' is meant enhancement of the degree of polarisation of the NMR-active nucleus over its' equilibrium polarisation. The natural abundance of ¹³C (relative to ¹²C) is about 1%, and suitable ¹³C-labelled compounds are suitably enriched to an abondance of at least 5%, preferably at least 50%, most preferably at least 90% before being hyperpolarised. At least one carbon atom of the metalloproteinase inhibitor of the present invention is suitably enriched with ¹³C, which is subsequently hyperpolarised.

When the imaging moiety is a reporter suitable for *in vivo* optical imaging, the reporter is any moiety capable of detection either directly or indirectly in an optical imaging procedure. The reporter might be a light scatterer (eg. a coloured or uncoloured particle), a light absorber or a light emitter. More preferably the reporter is a dye such as a chromophore or a fluorescent compound. The dye can be any dye that interacts with light in the electromagnetic spectrum with wavelengths from the ultraviolet light to the near infrared. Most preferably the reporter has fluorescent properties.

Preferred organic chromophoric and fluorophoric reporters include groups having an extensive delocalized electron system, eg. cyanines, merocyanines, indocyanines, phthalocyanines, naphthalocyanines, triphenylmethines, porphyrins, pyrilium dyes, thiapyriliup dyes, squarylium dyes, croconium dyes, azulenium dyes, indoanilines, benzophenoxazinium dyes, benzothiaphenothiazinium dyes, anthraquinones, napthoquinones, indathrenes, phthaloylacridones, trisphenoquinones, azo dyes, intramolecular and intermolecular charge-transfer dyes and dye complexes, tropones, tetrazines, bis(dithiolene) complexes, bis(benzene-dithiolate) complexes, iodoaniline dyes, bis(S,O-dithiolene) complexes. Fluorescent proteins, such as green fluorescent protein (GFP) and modifications of GFP that have different absorption/emission properties are also useful. Complexes of certain rare earth metals (e.g., europium, samarium, terbium or dysprosium) are used in certain contexts, as are fluorescent nanocrystals (quantum dots).

Particular examples of chromophores which may be used include: fluorescein, sulforhodamine 101 (Texas Red), rhodamine B, rhodamine 6G, rhodamine 19, indocyanine green, Cy2, Cy3, Cy3.5, Cy5, Cy5.5, Cy7, Marina Blue, Pacific Blue, Oregon Green 88, Oregon Green 514, tetramethylrhodamine, and Alexa Fluor 350, Alexa Fluor 430, Alexa Fluor 532, Alexa Fluor 546, Alexa Fluor 555, Alexa Fluor 568, Alexa Fluor 594, Alexa Fluor 633, Alexa Fluor 647, Alexa Fluor 660, Alexa Fluor 680, Alexa Fluor 700, and Alexa Fluor 750.

Particularly preferred are dyes which have absorption maxima in the visible or near infrared region, between 400 nm and 3 µm, particularly between 600 and 1300 nm.

Optical imaging modalities and measurement techniques include, but not limited to: luminescence imaging; endoscopy; fluorescence endoscopy; optical coherence tomography; transmittance imaging; time resolved transmittance imaging; confocal imaging; nonlinear microscopy; photoacoustic imaging; acousto-optical imaging; spectroscopy; reflectance spectroscopy; interferometry; coherence interferometry; diffuse optical tomography and fluorescence mediated diffuse optical tomography (continuous wave, time domain and frequency domain systems), and measurement of light scattering, absorption, polarisation, luminescence, fluorescence lifetime, quantum yield, and quenching.

When the imaging moiety is a β-emitter suitable for intravascular detection, suitable such *β*-emitters include the radiometals ⁶⁷Cu, ⁸⁹Sr, ⁹⁰Y, ¹⁵³Sm, ¹⁸⁶Re, ¹⁸⁸Re or ¹⁹²Ir, and the non-metals ³²P ³³P, ³⁸S, ³⁸Cl, ³⁹Cl, ⁸²Br and ⁸³Br.

The imaging moiety is preferably attached at the Y¹, Y², X³ or X¹/X² positions of the MMPi of Formula (I), and is most preferably attached at the Y¹ or Y² positions, with the Y¹ position being especially preferred when Y¹ is -(CH₂)_{w}-(C=O)-Z. It is especially preferred that the imaging moiety is attached to or comprises one of the R¹ or R² groups of a Y¹ = -(CH₂)_{w}- (C=O)-NR¹R² moiety.

The imaging agents of the present invention are preferably of Formula II: 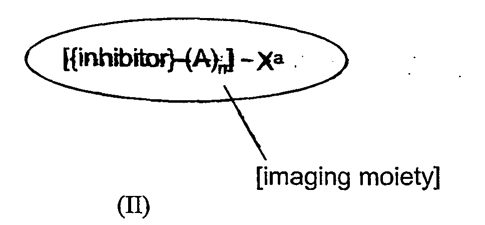 where:
{inhibitor} is the metalloproteinase inhibitor of Formula (I);
-(A)ₙ- is a linker group wherein each A is independently -CR₂-, -CR=CR- , -C≡C- , -CR₂CO₂- , -CO₂CR₂- , -NRCO- , -CONR- , -NR(C=O)NR-, -NR(C=S)NR-, -SO₂NR-, -NRSO₂-, -CR₂OCR₂- , -CR₂SCR₂-, -CR₂NRCR₂-, a C₄₋₈ cycloheteroalkylene group, a C₄₋₈ cycloalkylene group, a C₅₋₁₂ arylene group, or a C₃₋₁₂ heteroarylene group, an amino acid, a sugar or a monodisperse polyethyleneglycol (PEG) building block;
R is independently chosen from H, C₁₋₄ alkyl, C₂₄ alkenyl, C₂₋₄ alkynyl, C₁₋₄ alkoxyalkyl or C₁₋₄ hydroxyalkyl;
n is an integer of value 0 to 10,
and X^{a} is H, OH, Hal, NH₂, C₁₋₄ alkyl, C₁₋₄ alkoxy, C₁₋₄ alkoxyalkyl, C₁₋₄ hydroxyalkyl or X^{a} is the imaging moiety.

By the term "amino acid" is meant an L- or D-amino acid, amino acid analogue (eg. napthylalanine) or amino acid mimetic which may be naturally occurring or of purely synthetic origin, and may be optically pure, i.e. a single enantiomer and hence chiral, or a mixture of enantiomers. Preferably the amino acids of the present invention are optically pure.

By the term "sugar" is meant a mono-, di- or tri- saccharide. Suitable sugars include: glucose, galactose, maltose, mannose, and lactose. Optionally, the sugar may be functionalised to permit facile coupling to amino acids. Thus, eg. a glucosamine derivative of an amino acid can be conjugated to other amino acids *via* peptide bonds. The glucosamine derivative of asparagine (commercially available from Novabiochem) is one example of this:

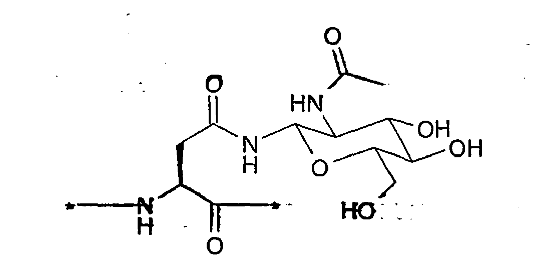

In Formula II, X^{a} is preferably the imaging moiety. This has the advantage that the linker group -(A)ₙ- of Formula II distances the imaging moiety from the active site of the metalloproteinase inhibitor. This is particularly important when the imaging moiety is relatively bulky (eg. a metal complex or a radioiodine atom), so that binding of the inhibitor to the MMP enzyme is not impaired. This can be achieved by a combination of flexibility (eg. simple alkyl chains), so that the bulky group has the freedom to position itself away from the active site and/or rigidity such as a cycloalkyl or aryl spacer which orientates the metal complex away from the active site.

The nature of the linker group can also be used to modify the biodistribution of the imaging agent. Thus, eg. the introduction of ether groups in the linker will help to minimise plasma protein binding. When -(A)ₙ- comprises a polyethyleneglycol (PEG) building block or a peptide chain of 1 to 10 amino acid residues, the linker group may function to modify the pharmacokinetics and blood clearance rates of the imaging agent *in vivo.* Such "biomodifier" linker groups may accelerate or reduce the clearance of the imaging agent from background tissue, such as muscle or liver, and/or from the blood, thus giving a better diagnostic image due to less background interference; when used to increase blood residence this is beneficial for maximising the probability of the imaging agent interacting with the targeting biomarker at the site of pathology. A biomodifier linker group may also be used to favour a particular route of excretion, eg. *via* the kidneys as opposed to *via* the liver.

When -(A)ₙ- comprises a peptide chain of 1 to 10 amino acid residues, the amino acid residues are preferably chosen from glycine, lysine, aspartic acid, glutamic acid or serine. When -(A)ₙ₋-comprises a PEG moiety, it preferably comprises units derived from oligomerisation of the monodisperse PEG-Uke structures of Formulae IIIA or IIIB: 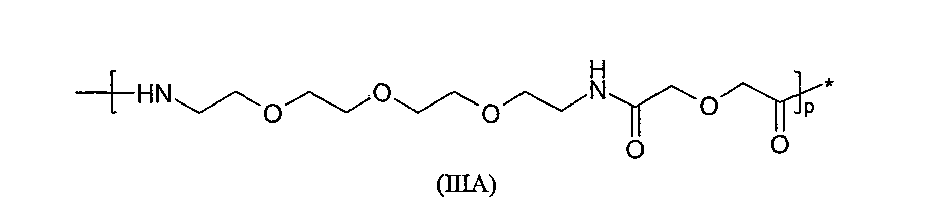 17-amino-5-oxo-6-aza-3, 9, 12, 15-tetraoxaheptadecanoic acid of Formula IIIA
wherein p is an integer from 1 to 10 and where the C-terminal unit (*) is connected to the imaging moiety. Alternatively, a PEG-like structure based on a propionic acid derivative of Formula IIIB can be used: 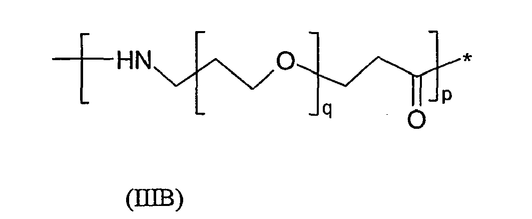 where p is as defined for Formula IIIA
and q is an integer from 3 to 15.

In Formula IIIB, p is preferably 1 or 2, and q is preferably 5 to 12.

When the linker group does not comprise PEG or a peptide chain, preferred -(A)ₙ-groups have a backbone chain of linked atoms which make up the -(A)ₙ moiety of 2 to 10 atoms, most preferably 2 to 5 atoms, with 2 or 3 atoms being especially preferred. A minimum linker group backbone chain of 2 atoms confers the advantage that the imaging moiety is well-separated from the metalloproteinase inhibitor so that any interaction is minimised.

Non-peptide linker groups such as alkylene groups or arylene groups have the advantage that there are no significant hydrogen bonding interactions with the conjugated MMP inhibitor, so that the linker does not wrap round onto the MMP inhibitor. Preferred alkylene spacer groups are -(CH₂)q- where q is 2 to 5. Preferred arylene spacers are of formular: 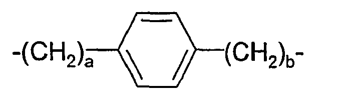 where: a and b are independently 0, 1 or 2.

The linker group -(A)ₙ- preferably comprises a diglycolic acid moiety, a maleimide moiety, a glutaric acid, succinic acid, a polyethyleneglycol based unit or a PEG-like unit of Formula IIIA.

When the imaging moiety comprises a metal ion, the metal ion is present as a metal complex. Such metalloproteinase inhibitor conjugates with metal ions are therefore suitably of Formula IIa: 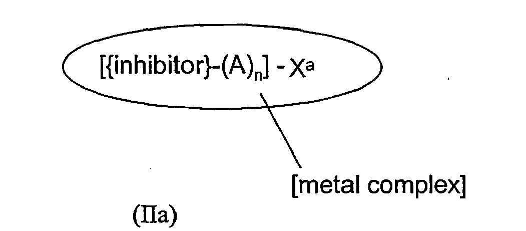 where: A, n and X^{a} are as defined for Formula II above.

By the term "metal complex" is meant a coordination complex of the metal ion with one or more ligands. It is strongly preferred that the metal complex is kinetically stable and hence "resistant to transchelation", ie. does not readily undergo ligand exchange with other potentially competing ligands for the metal coordination sites. Potentially competing ligands include the hydroxamic acid MMPi moiety itself plus other excipients in the preparation in *vitro* (eg. radioprotectants or antimicrobial preservatives used in the preparation), or endogenous compounds *in vivo* (eg. glutathione, transferrin or plasma proteins).

The metal complexes of Formula IIa are derived from conjugates of ligands of Formula IIb: 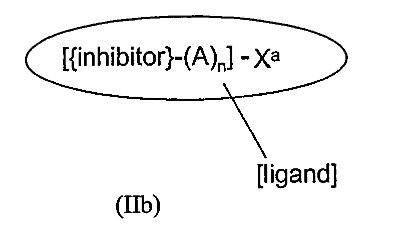 where: A, n and X^{a} are as defined for Formula II above.

Suitable ligands for use in the present invention which form metal complexes resistant to transchelation include: chelating agents, where 2-6, preferably 2-4, metal donor atoms are arranged such that 5- or 6-membered chelate rings result (by having a non-coordinating backbone of either carbon atoms or non-coordinating heteroatoms linking the metal donor atoms); or monodentate ligands which comprise donor atoms which bind strongly to the metal ion, such as isonitriles, phosphines or diazenides. Examples of donor atom types which bind well to metals as part of chelating agents are: amines, thiols, amides, oximes and phosphines. Phosphines form such strong metal complexes that even monodentate or bidentate phosphines form suitable metal complexes. The linear geometry of isonitriles and diazenides is such that they do not lend themselves readily to incorporation into chelating agents, and are hence typically used as monodentate ligands. Examples of suitable isonitriles include simple alkyl isonitriles such as *tert*-butylisonitrile, and ether-substituted isonitriles such as mibi (i.e. 1-isocyano-2-methoxy-2-methylpropane). Examples of suitable phosphines include Tetrofosmin, and monodentate phosphines such as tris(3-methoxypropyl)phosphine. Examples of suitable diazenides include the HYNIC series of ligands i.e. hydrazine-substituted pyridines or nicotinamides.

Preferred ligands are chelating agents, and monodentate ligands which form kinetically' stable metal complexes such as phosphines, isonitriles and diazenides. Most preferred ligands are chelating agents, as defined above.

Examples of suitable chelating agents for technetium which form metal complexes resistant to transchelation include, but are not limited to:
(i) diaminedioximes of formula: 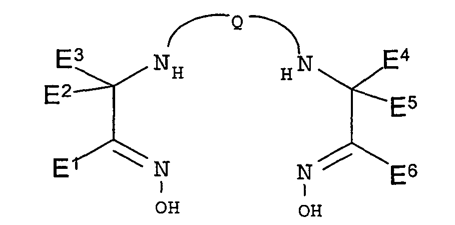 where E¹-E⁶ are each independently an R' group;
   each R' is H or C₁₋₁₀ alkyl, C₃₋₁₀ alkyl aryl, C₂₋₁₀ alkoxyalkyl, C₁₋₁₀ hydroxyalkyl, C₁₋₁₀ fluoroalkyl, C₂₋₁₀ carboxyalkyl or C₁₋₁₀ aminoalkyl, or two or more R' groups together with the atoms to which they are attached form a carbocyclic, heterocyclic, saturated or unsaturated ring, and wherein one or more of the R' groups is conjugated to the MMP inhibitor;
   and Q is a bridging group of formula -(J)_{f}-;
   where f is 3, 4 or 5 and each J is independently -O-, -NR'- or -C(R')₂- provided that - (J)_{f}-contains a maximum of one J group which is -O- or NR'-.
   Preferred Q groups are as follows:
   Q = -(CH₂)(CHR')(CH₂)- ie. propyleneamine oxime or PnAO derivatives;
   Q = -(CH₂)₂(CHR')(CH₂)₂- ie. pentyleneamine oxime or PentAO derivatives;
   Q = -(CH₂)₂NR'(CH₂)₂-.

   E¹ to E⁶ are preferably chosen from: C₁₋₃ alkyl, alkylaryl alkoxyalkyl, hydroxyalkyl, fluoroalkyl, carboxyalkyl or aminoalkyl. Most preferably, each E¹ to E⁶ group is CH₃.
   The MMP inhibitor is preferably conjugated at either the E¹ or E⁶ R' group, or an R' group of the Q moiety. Most preferably, the MMP inhibitor is conjugated to an R' group of the Q moiety. When the MMP inhibitor is conjugated to an R' group of the Q moiety, the R' group is preferably at the bridgehead position. In that case, Q is preferably -(CH₂)(CHR')(CH₂)-,
   -(CH₂)₂(CHR')(CH₂)₂- or -(CH₂)₂NR'(CH₂)₂-, most preferably -(CH₂)₂(CHR')(CH₂)₂-.
   An especially preferred bifunctional diaminedioxime chelator has the Formula: 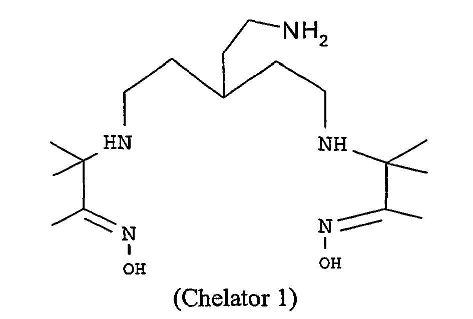 such that the MMP inhibitor is conjugated *via* the bridgehead -CH₂CH₂NH₂ group.
(ii) N₃S ligands having a thioltriamide donor set such as MAG₃ (mercaptoacetyltriglycine) and related ligands; or having a diamidepyridinethiol donor set such as Pica;
(iii) N₂S₂ ligands having a diaminedithiol donor set such as BAT or ECD (i.e. ethylcysteinate dimer), or an amideaminedithiol donor set such as MAMA;
(iv) N₄ ligands which are open chain or macrocyclic ligands having a tetramine, amidetriamine or diamidediamine donor set, such as cyclam, monoxocyclam or dioxocyclam.
(v) N₂O₂ ligands having a diaminediphenol donor set.

The above described ligands are particularly suitable for complexing technetium eg. ^{94m}Tc or ^{99m}Tc, and are described more fully by Jurisson et al [Chem.Rev., 99 2205-2218 (1999)]. The ligands are also useful for other metals, such as copper (⁶⁴Cu or ⁶⁷Cu), vanadium (eg. ⁴⁸V), iron (eg. ⁵²Fe), or cobalt (eg. ⁵⁵Co). Other suitable ligands are described in Sandoz WO 91/01144, which includes ligands which are particularly suitable for indium, yttrium and gadolinium, especially macrocyclic aminocarboxylate and aminophosphonic acid ligands. Ligands which form non-ionic (i.e. neutral) metal complexes of gadolinium are known and are described in US 4885363. When the radiometal ion is technetium, the ligand is preferably a chelating agent which is tetradentate. Preferred chelating agents for technetium are the diaminedioximes, or those having an N₂S₂ or N₃S donor set as described above.

Polydentate hydroxamic acids which are chelating agents are known to form metal complexes with radiometals, including ^{99m}Tc [Safavy et al, Bioconj. Chem., 4, 194-198 (1993)]. The present inventors have, however, found that monodentate hydroxamic acids such as when X³ is H in Formula (1), the hydroxamic acid MMPi may compete effectively with the conjugated ligand for the radiometal. Hence, when X³ is H particular care is needed in the selection of the ligand, ie. it is necessary to choose a ligand which competes effectively with the hydroxamic acid MMPi for the radiometal, to avoid formation of undesirable [hydroxamic acid]-[radiometal] metal complexes. Suitable such ligands include: phosphines; isonitriles; N4 chelating agents having a tetramine, amidetriamine or diamidediamine donor set; N₃S chelating agents having a thioltriamide donor or diamidepyridinethiol donor set; or N₂S₂ chelating agents having a diaminedithiol donor set such as BAT or an amideaminedithiol donor set such as MAMA. Preferred such ligands include: the N4, N₃S and N₂S₂ chelating agents described above, most preferably N4 tetramine and N2S2 diaminedithiol or diamidedithiol chelating agents, especially the N2S2 diaminedithiol chelator known as BAT:

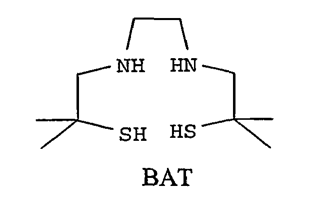

It is strongly preferred that the matrix metalloproteinase inhibitor is bound to the metal complex in such a way that the linkage does not undergo facile metabolism in blood, since that would result in the metal complex being cleaved off before the labelled metalloproteinase inhibitor reached the desired in *vivo* target site. The matrix metalloproteinase inhibitor is therefore preferably covalently bound to the metal complexes of the present invention *via* linkages which are not readily metabolised

When the imaging moiety is a radioactive halogen, such as iodine, the MMP inhibitor is suitably chosen to include: a non-radioactive halogen atom such as an aryl iodide or bromide (to permit radioiodine exchange); an activated aryl ring (e.g. a phenol group); an organometallic precursor compound (eg. trialkyltin or trialkylsilyl); an organic precursor such as triazenes or a good leaving group for nucleophilic substitution such as an iodonium salt. Methods of introducing radioactive halogens (including ¹²³I and ¹⁸F) are described by Bolton [J.Lab.Comp.Radiopharm., 45, 485-528 (2002)]. Examples of suitable aryl groups to which radioactive halogens, especially iodine can be attached are given below:

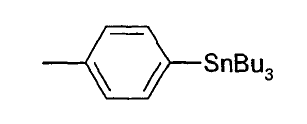

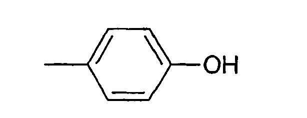

Both contain substituents which permit facile radioiodine substitution onto the aromatic ring. Alternative substituents containing radioactive iodine can be synthesised by direct iodination *via* radiohalogen exchange, e.g.

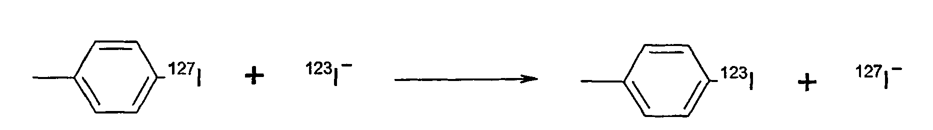

When the imaging moiety is a radioactive isotope of iodine the radioiodine atom is preferably attached *via* a direct covalent bond to an aromatic ring such as a benzene ring, or a vinyl group since it is known that iodine atoms bound to saturated aliphatic systems are prone to in *vivo* metabolism and hence loss of the radioiodine.

When the imaging moiety comprises a radioactive isotope of fluorine (eg. ¹⁸F), the radiohalogenation may be carried -out *via* direct labelling using the reaction of ¹⁸F-fluoride with a suitable precursor having a good leaving group, such as an alkyl bromide, alkyl mesylate or alkyl tosylate. ¹⁸F can also be introduced by N-alkylation of amine precursors with alkylating agents such as ¹⁸F(CH₂)₃OMs (where Ms is mesylate) to give N-(CH₂)₃¹⁸F, or O-alkylation of hydroxyl groups with ¹⁸F(CH₂)₃OMs or ¹⁸F(CH₂)₃Br. ¹⁸F can also be introduced by alkylation of N-haloacetyl groups with a ¹⁸ F(CH₂)₃OH reactant, to give -NH(CO)CH₂O(CH₂)₃¹⁸F derivatives. For aryl systems, ¹⁸F-fluoride nucleophilic displacement from an aryl diazonium salt, aryl nitro compound or an aryl quaternary ammonium salt are suitable routes to aryl-¹⁸F derivatives.

Primary amine-containing MMPis of Formula (I) can also be labelled with ¹⁸F by reductive amination using ¹⁸F-C₆H₄-CHO as taught by Kahn et al [J.Lab.Comp.Radiopharm. 45, 1045-1053 (2002)] and Borch et al [J. Am. Chem. Soc. 93, 2897 (1971)]. This approach can also usefully be applied to aryl primary amines, such as compounds comprising phenyl-NH₂ or phenyl-CH₂NH₂ groups.

Amine-containing MMP inhibitors of Formula (I) can also be labelled with ¹⁸F by reaction with ¹⁸F-labelled active esters such as: 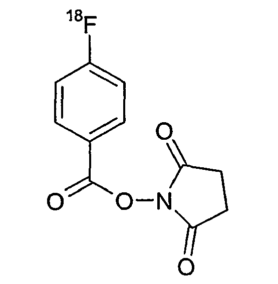 to give amide bond linked products. The N-hydroxysuccinimide ester shown and its use to label peptides is taught by Vaidyanathan et al [Nucl.Med.Biol., 19(3), 275-281 (1992)] and Johnstrom et al [Clin.Sci., 103 (Suppl. 48), 45-85 (2002)]. Further details of synthetic routes to ¹⁸F-labelled derivatives are described by Bolton, J.Lab.Comp.Radiopharm., 45, 485-528 (2002).

Introduction of PET radioisotope labels at the X³ position can be achieved by eg. O-alkylation of the corresponding hydroxamic acid derivative (X¹ = H) with triflate derivatives such as ¹¹CH₃OSO₂CF₃ as taught by Fei et al [J.Lab.Comp.Radiopharm., 46, 343-351 (2003)], or Zheng et al [Nucl.Med.Biol., 30, 753-760 (2003)], or the ¹⁸F O-alkylaling reagents described above. ¹¹C PET radiolabels can also be introduced by use of the above triflate derivative to alkylate phenolic hydroxyl groups as taught by Zheng et al [Nucl-Med Biol, 31, 77-85 (2004)]. Further methods of labelling with ¹¹C are taught by Antoni et al [Chapter 5 pages 141-194 in "Handbook of Radiopharmaceuticals", M.J. Welch and C.S. Redvanly (Eds.), Wiley (2003)].

Preferred matrix metalloproteinase inhibitors of the present invention are of Formula IV: 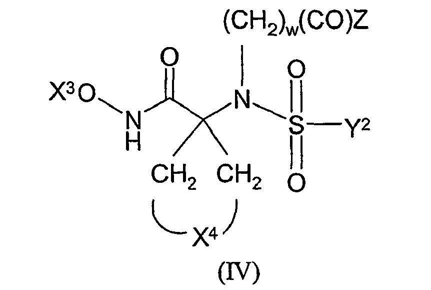 where:
Y², w and Z are as defined above;
X³ is H, CH₃ or CH₂F;
X⁴ is -(CH₂)ₘ- where m is 1, 2 or 3, -CH₂OCH₂ or X⁵ where X⁵ is 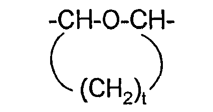
where t is 2 or 3.

In Formula (IV), X³ is preferably H or CH₃, most preferably H. X⁴ is preferably -(CH₂)₂-, -CH₂OCH₂- or an X⁶ group with t equal to 2. X⁴ is most preferably -(CH₂)₂- or -CH₂OCH₂-. Preferred Y², w and Z groups of Formula (IV) are as described for Formula (I) above.

When the imaging agent comprises a MMP inhibitor of Formula IV, and the imaging moiety is a gamma-emitting radioactive halogen, the imaging moiety is preferably attached at either the Y², Z or X⁴ substituents, most preferably the Y² or Z substituents. When the imaging moiety is a position-emitting radioactive non-metal, it is preferably attached at the X³, X⁴, Y² or Z substituents, most preferably the X⁴ or Z positions. When X³ is H, the positron-emitting radioactive non-metal is most preferably attached at the Z or X⁴ positions, most preferably the Z position.

When the imaging moiety is a radioactive or paramagnetic metal ion, one of the X⁴ or Z substituents is preferably attached to or comprises the imaging moiety. Most preferably, the Z substituent of Formula IV is preferably attached to or comprises the radioactive or paramagnetic metal ion imaging moiety.

Preferred matrix metalloproteinase inhibitors of the present invention are of Formula V: 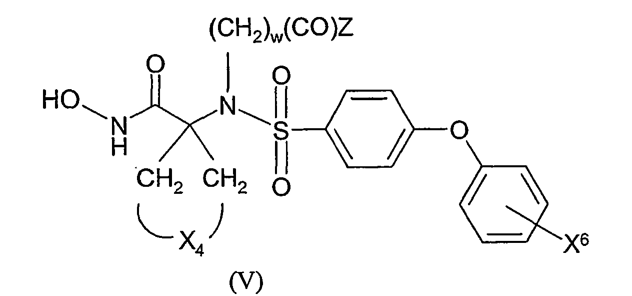 where X⁶ is Hal, R¹ or OR¹, where R¹ is C₁₋₃ alkyl or C₁₋₃ fluoroalkyl.

Preferred X⁴, w and Z groups of Formula (V) are as described for Formula (IV) above. w is most preferably 2. X⁶ is preferably F, most preferably 4-fluoro.

The MMP inhibitor compounds of the present invention may be prepared as summarised in Scheme 1 (overleaf)

The synthesis of the analogous MMPi Compound 27 is given in EP 0895988 A1 and Example 5. Further references to syntheses are provided in the review by Skiles et al [Curr.Med.Chem., 8 425-474 (2001)].

When the imaging agent of the present invention comprises a radioactive or paramagnetic metal ion, the metal ion is suitably present as a metal complex Such metal complexes are suitably prepared by reaction of the conjugate of Formula IIb with the appropriate metal ion. The ligand-conjugate or chelator-congugate of the MMP inhibitor of Formula IIb can be prepared *via* the bifunctional chelate approach. Thus, it is well known to prepare ligands or chelating agents which have attached thereto a functional group ("bifunctional linkers" or "bifunctional chelates" respectively). Functional groups that have been attached include: amine, thiocyanate, 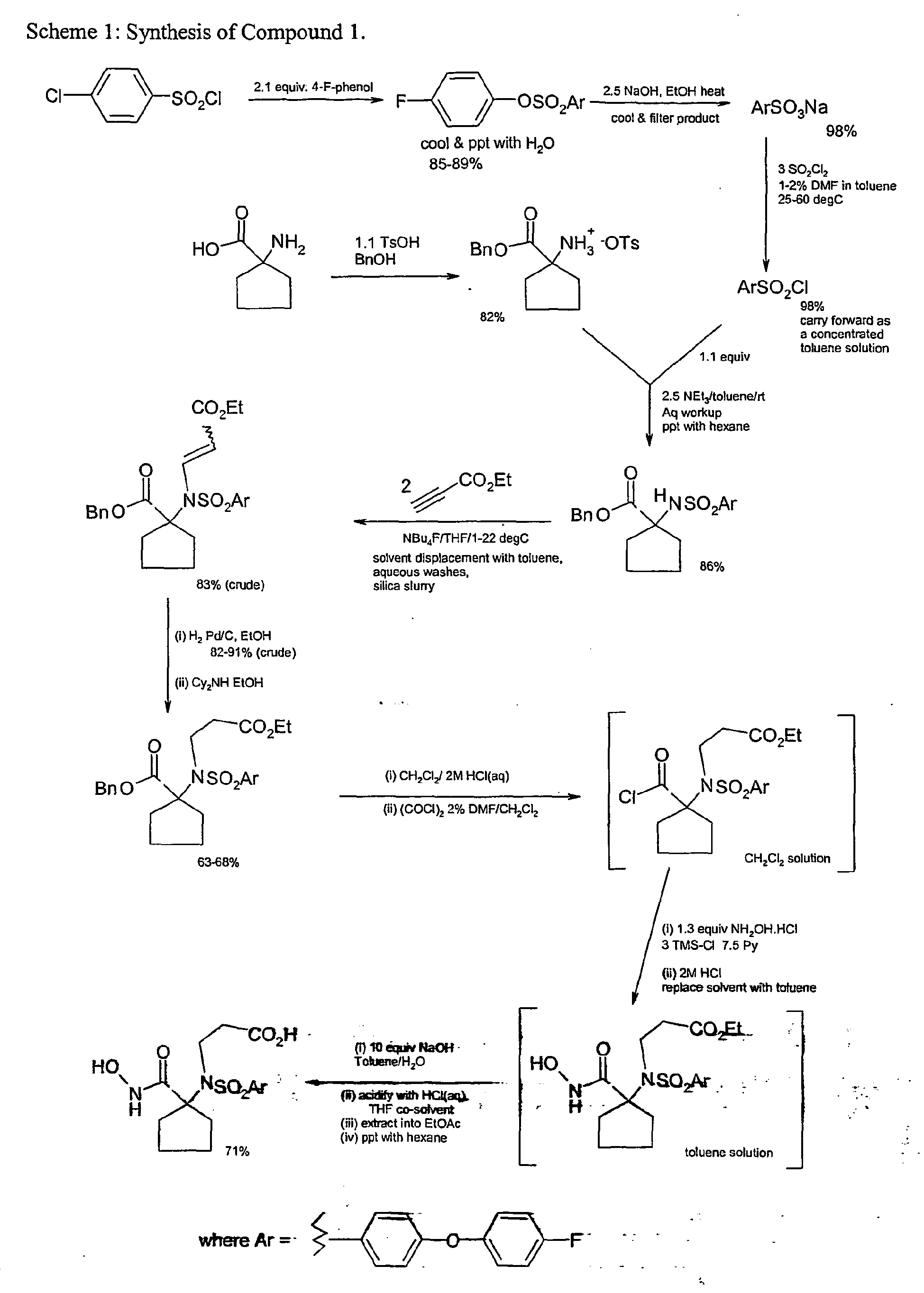 maleimide and active esters such as N-hydroxysuccinimide or pentafluorophenol. Chelator 1 of the present invention is an example of an amine-functionalised bifunctional chelate. Bifunctional chelates based on thiolactones, which can be used to prepare BAT chelator-conjugates are described by Baidoo et al [Bioconj. Chem., 5, 114-118 (1994)]. Bifunctional chelates suitable for complexation to a technetium or rhenium tricarbonyl core are described by Stichelberger *et.al* [Versatile synthetic approach to new bifunctional chelating agents tailor made for labeling with the fac-[M(CO)₃]⁺ core (M = Tc, ^{99m}Tc, Re): synthesis, in *vitro,* and *in vivo* behavior of the model complex [M(APPA)(CO)₃] (appa = [(5-amino-pentyl)-pyridin-2-yl-methyl-amino]-acetic acid); Nucl. Med. Biol., 30, 465-470 (2003)]. Bifunctional HYNIC ligands are described by Edwards et al [Bioconj.Chem., 8, 146 (1997)]. Such bifunctional chelates can be reacted with suitable functional groups on the matrix metalloproteinase inhibitor to form the desired conjugate. Such suitable functional groups on the inhibitor include:
carboxyls (for amide bond formation with an amine-functionalised bifunctional chelator); amines (for amide bond formation with an carboxyl- or active ester-functionalised bifunctional chelator);
halogens, mesylates and tosylates (for N-alkylation of an amine-functionalised bifunctional chelator) and
thiols (for reaction with a maleimide-functionalised bifunctional chelator).

The radiolabelling of the MMP inhibitors of the present invention can be conveniently carried out using "precursors". When the imaging moiety comprises a metal ion, such precursors suitably comprise "conjugates" of the MMP inhibitor with a ligand, as described in the fourth embodiment below. When the imaging moiety comprises a non-metallic radioisotope, ie. a gamma-emitting radioactive halogen or a positron-emitting radioactive non-metal, such "precursors" suitably comprise a non-radioactive material which is designed so that chemical reaction with a convenient chemical form of the desired non-metallic radioisotope can be conducted in the minimum number of steps (ideally a single step), and without the need for significant purification (ideally no further purification) to give the desired radioactive product. Such precursors can conveniently be obtained in good chemical purity and, optionally supplied in sterile form.

It is envisaged that "precursors" (including ligand conjugates) for radiolabelling of the MMP inhibitors of the present invention can be prepared as follows:

The terminal -OH group of an -N(CH₂)₂OH or -N(CH₂)₃OH derivative may be converted to a tosyl or mesyl group or bromo derivative, which can then be used to conjugate an amino-functionalised chelator. Such tosylate, mesylate or bromo groups of the precursors described may alternatively be displaced with [¹⁸F]fluoride to give an ¹⁸F-labelled PET imaging agent.

Radioiodine derivatives can be prepared from the corresponding phenol precursors. Alkyl bromide derivatives may be used for N-alkylation of an amine-functionalised chelator. Phenyl iodide derivatives can be converted to organometallic precursors for radioiodination compounds, such as trialkyltin or aryl trimethylsilyl (TMS) precursors. Phenyl iodide derivatives can also be converted to an aryl iodonium precursor for radiofluorination with ¹⁸F-fluoride.

Primary amine-functionalised MMP inhibitors may be reacted with acid anhydrides to give N-functionalised precursors of the type -N(CO)(CH₂)₃CO₂H, which can then be conjugated to bifunctional amine-containing ligands. Such primary amine substituted MMPis can be prepared by alkylation of bromo derivatives with benzylamine, followed by removal of the benzyl protecting group under standard conditions such as hydrogenation using a palladium catalyst on charcoal.

Amine-functionalised MMPis may be conjugated directly with a carboxyl- or active ester-functionalised bifunctional chelator, or *via* a linker. Such compounds may also be reacted with a alkylating agent suitable for ¹⁸F labelling such as ¹⁸F(CH₂)₂OTs (where Ts is a tosylate group) or ¹⁸F(CH₂)₂OMs (where Ms is a mesylate group), to give the corresponding N-functionalised amine derivative having an N(CH₂)₂¹⁸F substituent. Alternatively, the amine can first be reacted with chloroacetyl chloride to give the -N(CO)CH₂Cl N-derivatised amide, followed by reaction with HS(CH₂)₃¹⁸F or HO(CH₂)₃¹⁸F to give the -N(CO)CH₂S(CH₂)₃¹⁸F and -N(CO)CH₂O(CH₂)₃¹⁸F products respectively.

The radiometal complexes of the present invention may be prepared by reaction of a solution of the radiometal in the appropriate oxidation state with the ligand conjugate of Formula IIa at the appropriate pH. The solution may preferably contain a ligand which complexes weakly to the metal (such as gluconate or citrate) i.e. the radiometal complex is prepared by ligand exchange or transchelation. Such conditions are useful to suppress undesirable side reactions such as hydrolysis of the metal ion. When the radiometal ion is ^{99m}Tc, the usual starting material is sodium pertechnetate from a ⁹⁹Mo generator. Technetium is present in ^{99m}Tc-pertechnetate in the Tc(VII) oxidation state, which is relatively unreactive. The preparation of technetium complexes of lower oxidation state Tc(I) to Tc(V) therefore usually requires the addition of a suitable pharmaceutically acceptable reducing agent such as sodium dithionite, sodium bisulphite, ascorbic acid, formamidine sulphinic acid, stannous ion, Fe(II) or Cu(I), to facilitate complexation. The pharmaceutically acceptable reducing agent is preferably a stannous salt, most preferably stannous chloride, stannous fluoride or stannous tartrate.

When the imaging moiety is a hyperpolarised NMR-active nucleus, such as a hyperpolarised ¹³C atom, the desired hyperpolarised compound can be prepared by polarisation exchange from a hyperpolarised gas (such as ¹²⁹Xe or ³He) to a suitable ¹³C-enriched hydroxamic acid derivative.

In a second aspect, the present invention provides a pharmaceutical composition which comprises the imaging agent as described above, together with a biocompatible carrier, in a form suitable for mammalian administration. The "biocompatible carrier" is a fluid, especially a liquid, which in which the imaging agent can be suspended or dissolved, such that the composition is physiologically tolerable, ie. can be administered to the mammalian body without toxicity or undue discomfort. The biocompatible carrier is suitably an injectable carrier liquid such as sterile, pyrogen-free water for injection; an aqueous solution such as saline (which may advantageously be balanced so that the final product for injection is either isotonic or not hypotonic); an aqueous solution of one or more tonicity-adjusting substances (eg. salts of plasma cations with biocompatible counterions), sugars (e.g. glucose or sucrose), sugar alcohols (eg. sorbitol or mannitol), glycols (eg. glycerol), or other non ionic polyol materials (eg. polyethyleneglycols, propylene glycols and the like).

In a third aspect, the present invention provides a radiopharmaceutical composition which comprises the imaging agent as described above wherein the imaging moiety is radioactive, together with a biocompatible carrier (as defined above), in a form suitable for mammalian administration. Such radiopharmaceuticals are suitably supplied in either a container which is provided with a seal which is suitable for single or multiple puncturing with a hypodermic needle (e.g. a crimped-on septum seal closure) whilst maintaining sterile integrity. Such containers may contain single or multiple patient doses. Preferred multiple dose containers comprise a single bulk vial (e.g. of 10 to 30 cm³ volume) which contains multiple patient doses, whereby single patient doses can thus be withdrawn into clinical grade syringes at various time intervals during the viable lifetime of the preparation to suit the clinical situation. Pre-filled syringes are designed to contain a single human dose, and are therefore preferably a disposable or other syringe suitable for clinical use. The pre-filled syringe may optionally be provided with a syringe shield to protect the operator from radioactive dose. Suitable such radiopharmaceutical syringe shields are known in the art and preferably comprise either lead or tungsten.

When the imaging moiety comprises ^{99m}Tc, a radioactivity content suitable for a diagnostic imaging radiopharmaceutical is in the range 180 to 1500 MBq of ^{99m}Tc, depending on the site to be imaged in *vivo*, the uptake and the target to background ratio.

The radiopharmaceuticals of the present invention may be prepared from kits, as is described in the fifth and sixth embodiments below. Alternatively, the radiopharmaceuticals may be prepared under aseptic manufacture conditions to give the desired sterile product. The radiopharmaceuticals may also be prepared under non-sterile conditions, followed by terminal sterilisation using e.g. gamma-irradiation, autoclaving, dry heat or-chemical treatment (e.g. with ethylene oxide). Preferably, the radiopharmaceuticals of the present invention are prepared from kits.

In a fourth aspect, the present invention provides a conjugate of the matrix metalloproteinase inhibitor of Formula (I) with a ligand. Said ligand conjugates are useful for the preparation of matrix metalloproteinase inhibitors labelled with either a radioactive metal ion or a paramagnetic metal ion. Preferably, the ligand conjugate is of Formula IIa, as defined above. Most preferably, the MMP inhibitor of the ligand conjugate is of Formula IV, as defined above. The ligand of the conjugate of the fourth aspect of the invention is preferably a chelating agent. Preferably, the chelating agent has a diaminedioxime, N₂S₂, or N₃S donor set.

In a fifth aspect, the present invention provides a non-radioactive kit for the preparation of the radiopharmaceutical composition described above where the imaging moiety comprises a radiometal, which comprises a conjugate of a ligand with the matrix metalloproteinase inhibitor of Formula (I). When the radiometal is ^{99m}Tc, the kit suitably further comprises a biocompatible reductant. The ligand conjugates, and preferred aspects thereof, are described in the fourth embodiment above.

Such kits are designed to give sterile radiopharmaceutical products suitable for human administration, e.g. *via* direct injection into the bloodstream. For ^{99m}Tc, the kit is preferably lyophilised and is designed to be reconstituted with sterile ^{99m}Tc-pertechnetate (TcO₄⁻) from a ^{99m}Tc radioisotope generator to give a solution suitable for human administration without further manipulation. Suitable kits comprise a sealed container which permits maintenance of sterile integrity and/or radioactive safety, plus optionally an inert headspace gas (eg. nitrogen or argon), whilst permitting addition and withdrawal of solutions by syringe. A preferred such container is a septum-sealed vial, wherein the gas-tight closure is crimped on with an overseal (typically of aluminium). Such containers have the additional advantage that the closure can withstand vacuum if desired eg. to change the headspace gas or degas solutions. The kit comprises the ligand or chelator conjugate in either free base or acid salt form, together with a biocompatible reductant such as sodium dithionite, sodium bisulphite, ascorbic acid, formamidine sulphinic acid, stannous ion, Fe(II) or Cu(I). The biocompatible reductant is preferably a stannous salt such as stannous chloride or starmous tartrate. Alternatively, the kit may optionally contain a metal complex which, upon addition of the radiometal, undergoes transmetallation (i.e. metal exchange) giving the desired product.

The non-radioactive kits may optionally further comprise additional components such as a transchelator, radioprotectant, antimicrobial preservative, pH-adjusting agent or filler. The "transchelator" is a compound which reacts rapidly to form a weak complex with technetium, then is displaced by the ligand. This minimises the risk of formation of reduced hydrolysed technetium (RHT) due to rapid reduction of pertechnetate competing with technetium complexation. Suitable such transchelators are salts of a weak organic acid, ie. an organic acid having a pKa in the range 3 to 7, with a biocompatible cation. Suitable such weak organic acids are acetic acid, citric acid, tartaric acid, gluconic acid, glucoheptonic acid, benzoic acid, phenols or phosphonic acids. Hence, suitable salts are acetates, citrates, tartrates, gluconates, glucoheptonates, benzoates, phenolates or phosphonates. Preferred such salts are tartrates, gluconates, glucoheptonates, benzoates, or phosphonates, most preferably phosphonates, most especially diphosphonates. A preferred such transchelator is a salt of MDP, ie. methylenediphosphonic acid, with a biocompatible cation. By the term "biocompatible cation" is meant a positively charged counterion which forms a salt with an ionised, negatively charged anionic group, where said positively charged counterion is also non-toxic and hence suitable for administration to the mammalian body, especially the human body. Examples of suitable biocompatible cations include: the alkali metals sodium or potassium; the alkaline earth metals calcium and magnesium; and the ammonium ion. Preferred biocompatible cations are sodium and potassium, most preferably sodium.

By the term "radioprotectant" is meant a compound which inhibits degradation reactions, such as redox processes, by trapping highly-reactive free radicals, such as oxygen-containing free radicals arising from the radiolysis of water. The radioprotectants of the present invention are suitably chosen from: ascorbic acid, para-aminobenzoic acid (ie. 4-aminobenzoic acid), gentisic acid (ie. 2,5-dihydroxybenzoic acid) and salts thereof with a biocompatible cation as described above.

By the term "antimicrobial preservative" is meant an agent which inhibits the growth of potentially harmful: micro-organisms such as bacteria, yeasts or moulds. The antimicrobial preservative may also exhibit some bactericidal properties, depending on the dose. The main role of the antimicrobial preservative(s) of the present invention is to inhibit the growth of any such micro-organism in the radiopharmaceutical composition post-reconstitution, ie. in the radioactive diagnostic product itself The antimicrobial preservative may, however, also optionally be used to inhibit the growth of potentially harmful micro-organisms in one or more components of the non-radioactive kit of the present invention prior to reconstitution. Suitable antimicrobial preservative(s) include: the parabens, ie. methyl, ethyl, propyl or butyl paraben or mixtures thereof; benzyl alcohol; phenol; cresol; cetrimide and thiomersal. Preferred antimicrobial preservative(s) are the parabens.

The term "pH-adjusting agent" means a compound or mixture of compounds useful to ensure that the pH of the reconstituted kit is within acceptable limits (approximately pH 4.0 to 10.5) for human or mammalian administration. Suitable such pH-adjusting agents include pharmaceutically acceptable buffers, such as tricine, phosphate or TRIS [ie. *tris*(hydroxymethyl)aminomethane], and pharmaceutically acceptable bases such as sodium carbonate, sodium bicarbonate or mixtures thereof. When the conjugate is employed in acid salt form, the pH adjusting agent may optionally be provided in a separate vial or container, so that the user of the kit can adjust the pH as part of a multi-step procedure.

By the term "filler" is meant a pharmaceutically acceptable bulking agent which may facilitate material handling during production and lyophilisation. Suitable fillers include inorganic salts such as sodium chloride, and water soluble sugars or sugar alcohols such as sucrose, maltose, mannitol or trehalose.

In a sixth aspect, the present invention provides kits for the preparation of radiopharmaceutical preparations where the imaging moiety comprises a non-metallic radioisotope, ie. a gamma-emitting radioactive halogen or a positron-emitting radioactive non-metal. Such kits comprise a "precursor"- as described below, preferably in sterile non-pyrogenic form, so that reaction with a sterile source of the radioisotope gives the desired radiopharmaceutical with the minimum number of manipulations. Such considerations are particularly important for radiopharmaceuticals where the radioisotope has a relatively short half-life, and for ease of handling and hence reduced radiation dose for the radiopharmacist. Hence, the reaction medium for reconstitution of such kits is preferably aqueous, and in a form suitable for mammalian administration. The precursor is preferably provided in a sealed container, as described for the fourth embodiment above.

The "precursor" suitably comprises a non-radioactive derivative of the matrix metalloproteinase inhibitor material in sterile, apyrogenic form, which is designed so that chemical reaction with a convenient chemical form of the desired non-metallic radioisotope can be conducted in the minimum number of steps (ideally a single step), and without the need for significant purification (ideally no further purification) to give the desired radioactive product. Such precursors can conveniently be obtained in good chemical purity. Suitable precursors are derived from examples described in Bolton, J.Lab.Comp.Radiopharm., 45, 485-528 (2002).

Preferred precursors of this embodiment comprise a derivative which either undergoes electrophilic or nucleophilic halogenation; undergoes facile alkylation with an alkylating agent chosen from an alkyl or fluoroalkyl halide, tosylate, triflate (ie. trifluoromethanesulphonate) or mesylate; or alkylates thiol moieties to form thioether linkages. Examples of the first category are:
(a) organometallic derivatives such as a trialkylstannane (eg. trimethylstannyl or tributylstannyl), or a trialkylsilane (eg. trimethylsilyl);
(b) a non-radioactive alkyl iodide or alkyl bromide for halogen exchange and alkyl tosylate, mesylate or triflate for nucleophilic halogenation;
(c) aromatic rings activated towards electrophilic halogenation (eg. phenols) and aromatic rings activated towards nucleophilic halogenation (eg. aryl iodonium, aryl diazonium, nitroaryl).

Preferred derivatives which undergo facile alkylation are alcohols, phenols or amine groups, especially phenols and sterically-unhindered primary or secondary amines.

Preferred derivatives which alkylate thiol-containing radioisotope reactants are N-haloacetyl groups, especially N-chloroacetyl and N-bromoacetyl derivatives.

The precursors may be employed under aseptic manufacture conditions to give the desired sterile, non-pyrogenic material. The precursors may also be employed under non-sterile conditions, followed by terminal sterilisation using e.g. gamma-irradiation, autoclaving, dry heat or chemical treatment (e.g. with ethylene oxide). Preferably, the precursors are employed in sterile, non-pyrogenic form.

When X³ in Formula I is H, suitable precursors for MMPi's of Formula I may therefore comprise a derivative where X³ is a protecting group (P^{G}) for the hydroxamic acid moiety. By the term "protecting group" is meant a group which inhibits or suppresses undesirable chemical reactions, but which is designed to be sufficiently reactive that it may be cleaved from the functional group in question under mild enough conditions that do not modify the rest of the molecule. After deprotection the desired product is obtained. Protecting groups are well known to those skilled in the art and are suitably chosen from, for amine groups: Boc (where Boc is tert-butyloxycarbonyl), Fmoc (where Fmoc is fluorenylmethoxycarbonyl), trifluoroacetyl, allyloxycarbonyl, Dde [i.e. 1-(4,4-dimethyl-2,6-dioxocyclohexylidene)ethyl] or Npys (i.e. 3-nitro-2-pyridine sulfenyl); and for carboxyl groups: methyl ester, tert-butyl ester or benzyl ester. For hydroxyl groups, suitable protecting groups are: benzyl, acetyl, benzoyl, trityl (Trt) or trialkylsilyl such as tetrabutyldimethylsilyl. For thiol groups, suitable protecting groups are: trityl and 4-methoxybenzyl. Preferred protecting groups for the hydroxyl group of a hydroxamic acid moiety are: benzyl or trialkylsilyl. The use of further protecting groups are described in 'Protective Groups in Organic Synthesis', Theorodora W. Greene and Peter G. M. Wuts, (John Wiley & Sons, 1991).

Preferred convenient chemical forms of the desired non-metallic radioisotope include:
(a) halide ions (eg. ¹²³*-*I-iodide or ¹⁸F-fluoride), especially in aqueous media, for substitution reactions;
(b) ¹¹C-methyl iodide or ¹⁸F-fluoroalkylene compounds having a good leaving group, such as bromide, mesylate or tosylate;
(c) HS(CH₂)₃¹⁸F for S-alkylation reactions with alkylating precursors such as N-chloroacetyl or N-bromoacetyl derivatives.

Examples of suitable such "precursors", and methods for their preparation are described in the first embodiment (above).

The "precursor" of the kit is preferably supplied covalently attached to a solid support matrix. In that way, the desired radiopharmaceutical product forms in solution, whereas starting materials and impurities remain bound to the solid phase. Precursors for solid phase electrophilic fluorination with ¹⁸F-fluoride are described in WO 03/002489. Precursors for solid phase nucleophilic fluorination with ¹⁸F-fluoride are described in WO 03/002157. The kit may therefore contain a cartridge which can be plugged into a suitably adapted automated synthesizer. The cartridge may contain, apart from the solid support- bound precursor, a column to remove unwanted fluoride ion, and an appropriate vessel connected so as to allow the reaction mixture to be evaporated and allow the product to be formulated as required. The reagents and solvents and other consumables required for the synthesis may also be included together with a compact disc carrying the software which allows the synthesiser to be operated in a way so as to meet the customer requirements for radioactive concentration, volumes, time of delivery etc. Conveniently, all components of the kit are disposable to minimise the possibility of contamination between runs and will be sterile and quality assured.

In an eighth aspect, the present invention discloses the use of the matrix metalloproteinase inhibitor imaging agent described above for the diagnostic imaging of atherosclerosis, especially unstable vulnerable plaques.

In a further aspect, the present invention discloses the use of the matrix metalloproteinase inhibitor imaging agent described above for the diagnostic imaging of other inflammatory diseases, cancer, or degenerative diseases.

In a further aspect, the present invention discloses the use of the matrix metalloproteinase inhibitor imaging agent described above for the intravascular detection of atherosclerosis, especially unstable vulnerable plaques, using proximity detection. Such proximity detection may be achieved using intravascular device such as catheters or intra-operatively using hand-held detectors (eg. gamma detectors). Such intravascular detection isparticularly useful When the imaging moiety is a reporter group suitable for *in vivo* optical imaging or a β-emitter, since such moieties may not be readily detected outside the mammalian body, but are suitable for proximity detection.

The invention is illustrated by the non-limiting Examples detailed below. Example 1 describes the synthesis of the compound 1,1,1-*tris*(2-aminoethyl)methane. Example 2 provides an alternative synthesis of 1,1,1-*tris*(2-aminoethyl)methane which avoids the use of potentially hazardous azide intermediates. Example 3 describes the synthesis of a chloronitrosoalkane precursor. Example 4 describes the synthesis of a preferred amine-substituted bifunctional diaminedioxime of the present invention (Chelator 1).

Example 5 provides a synthesis of a MMPi of the invention, Compound 27. Example 6 provides the synthesis of a phenol-substituted MMPi precursor suitable for radiohalogenation (Compound 23). Example 7 describes the synthesis of an iodoaniline precursor suitable for radiohalogenation (Compound 26). Example 9 provides the synthesis of a chelator conjugate of an MMPi of the invention. Example 10 provides the synthesis of an MMPi functionalised with a PEG linker group. Example 11 describes the synthesis of a chelator conjugate having a PEG linker group. Example 12 provides the synthesis of a chloroacetyl precursor suitable for PET radiolabelling. Example 13 provides the synthesis of thioether-linked fluoroalkyl MMPi derivatives. Example 14 provides a range of amino acid and/or PEG-linked MMPis to permit modification of the biological properties.

Examples 15 and 16 provide the syntheses of suitable ¹⁸F-labelled compounds for ¹⁸F MMPi radiolabelling. Example 17 provides the synthesis of compounds 45 to 48. Example 18 describes *in vitro* assays showing that the derivatives of the MMPis of the present invention retain biological activity as MMP inhibitors. Example 19 provides a general ^{99m}Tc radiolabelling method for chelator conjugates. Example 20 provides a radioiodination procedure for suitable precursors of the invention. Example 21 provides a preparation of specific ¹⁸F derivatives of the invention. Example 22 provides evidence that radioiodinated derivatives of the invention exhibit adequate plasma stability to function as *in vivo* imaging agents. Example 23 describes the uptake of radioiodinated imaging agents of the invention in tumour models *in vivo.* This shows that the biodistribution can be modified using the linker groups of the invention. Compound 20A (ie. Compound 24A with a PEG3 spacer) showed similar blood residence, but a 10% increase in urinary excretion and corresponding 10% decrease in HBS compared to Compound 24A. Therefore the addition of a biomodifier resulted in a change in pharmacokinetics. Uptake into the tumour was slightly lower than that seen for Compound 24A but retention was slightly increased. Compound 32A exhibited high initial blood retention, which cleared with time. Good tumour uptake and retention were seen up to 1 hour post injection. High urinary excretion and low GI excretion were seen. These pharmacokinetics are more desirable and significantly different from those of the compound without the biomodifier (ie. Compound 24A), demonstrating the beneficial effects of biomodification with these compounds with no loss of inhibition potency.

Example 24 describes the uptake of ¹⁸F-labelled imaging agents of the invention in tumour models *in vivo.* Example 25 describes the uptake of imaging agents of the invention in an *in vivo* model of atherosclerosis. Example 26 provides autoradiography evidence that the agents of the invention are taken up at sites of atherosclerosis *in vivo.* Example 27 describes tumour imaging in a tumour models.

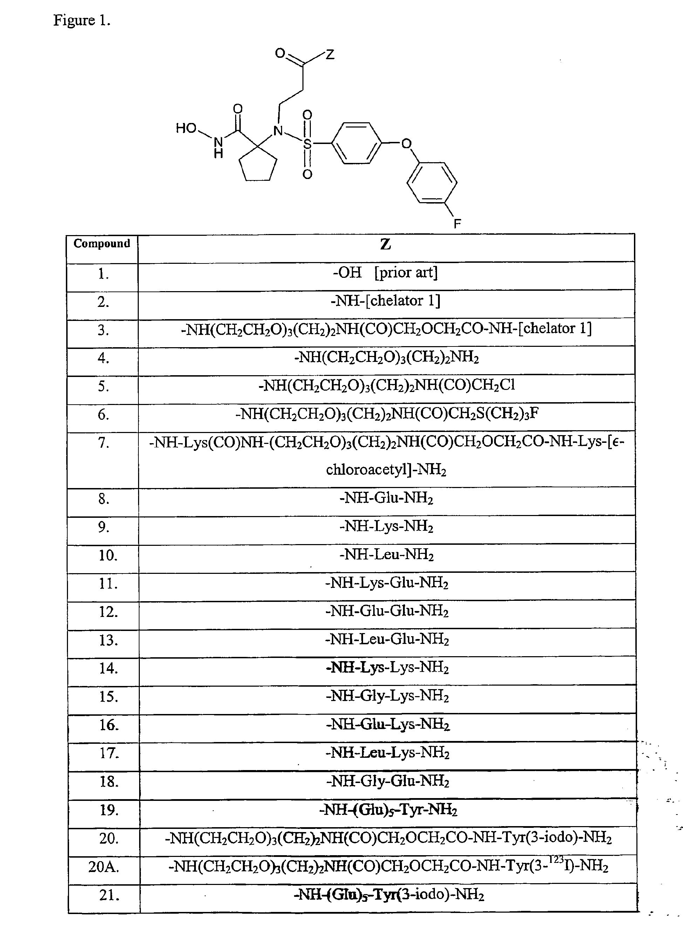

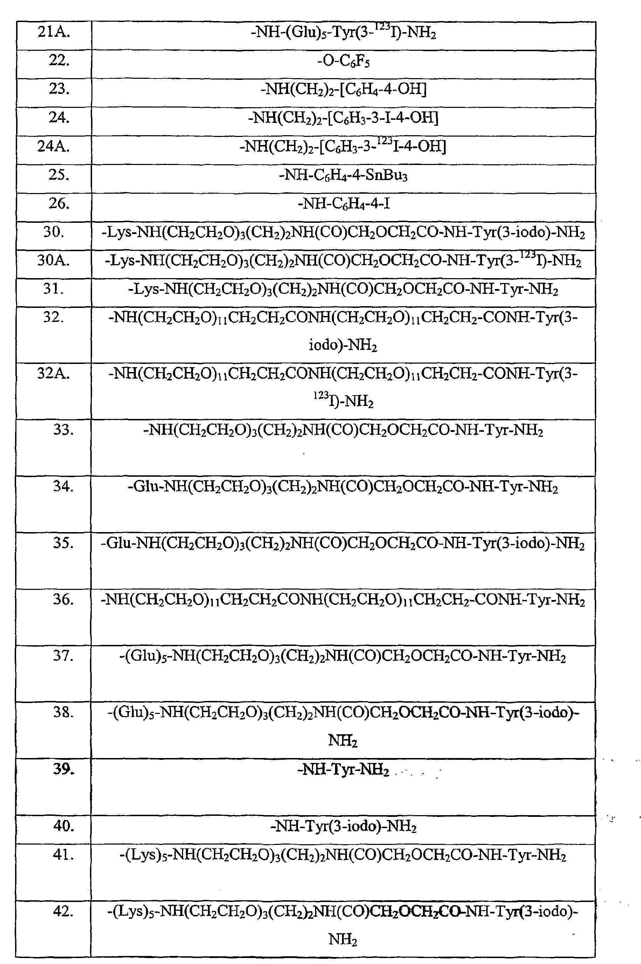

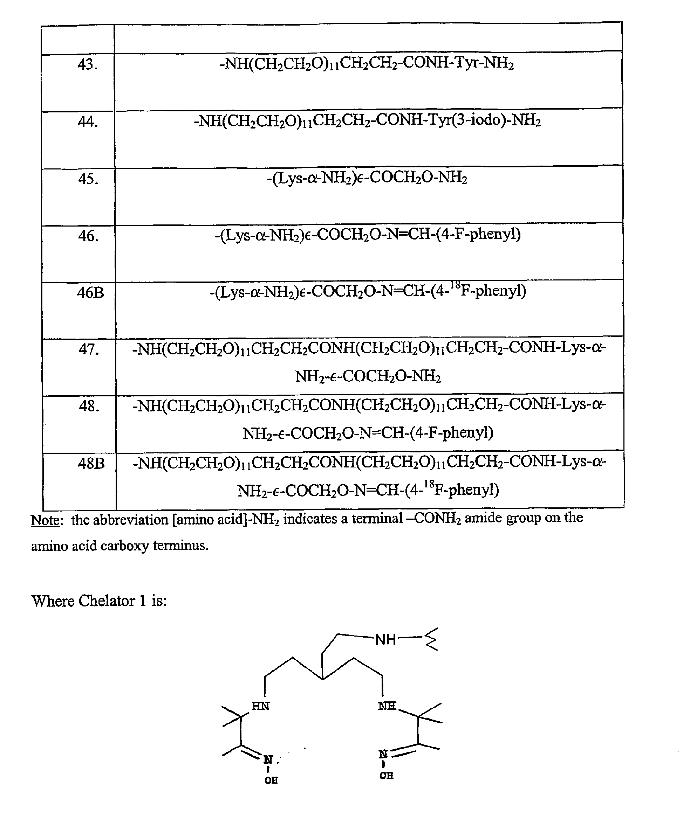

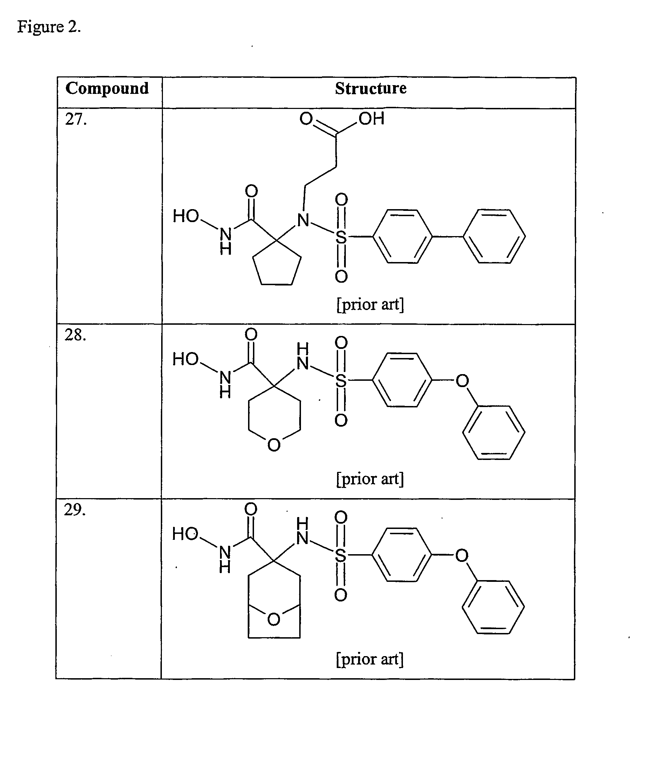

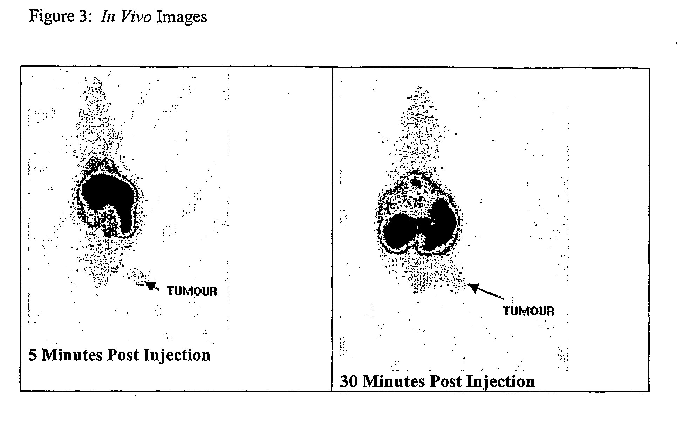

Figure 1 shows the chemical structures of several compounds of the invention, including an MMPi from which they are derived (Compound 1). Figure 2 shows the chemical structures of 3 MMPis of the invention. Figure 3 shows images obtained from Example 27.

### Example 1 : Synthesis of 1,1,1- tris(2-aminoethyl)methane.

### (Step a): 3-(methoxycarbonylmethylene)glutaric acid dimethylester.

Carbomethoxymethylenetriphenylphosphorane (167g, 0.5mol) in toluene (600ml) was treated with dimethyl 3-oxoglutarate (87g, 0.5mol) and the reaction heated to 100°C on an oil bath at 120°C under an atmosphere of nitrogen for 36h. The reaction was then concentrated *in vacuo* and the oily residue triturated with 40/60 petrol ether/diethylether 1:1,600ml. Triphenylphosphine oxide precipitated out and the supernatant liquid was decanted/filtered off The residue on evaporation *in vacuo* was Kugelrohr distilled under high vacuum Bpt (oven temperature 180-200°C at 0.2torr) to give 3-(methoxycarbonylmethylene)glutaric acid dimethylester (89.08g, 53%).
NMR ¹H(CDCl₃): δ 331 (2H, s, CH₂), 3.7 (9H, s, 3×OCH₃), 3.87 (2H, s, CH₂), 5.79 (1H, s, =CH,) ppm.
NMR ¹³C(CDCl₃), δ 36.56,CH₃, 48.7, 2xCH₃, 52.09 and 52.5 (2xCH₂); 122.3 and 146.16 C=CH; 165.9, 170.0 and 170.5 3xCOO ppm.

### (Step b): Hydrogenation of 3-(methoxycarbonylmethylene)glutaric acid dimethylester.

3-(methoxycarbonylmethylene)glutaric acid dimethylester (89g, 267mmol) in methanol (200ml) was shaken with (10% palladium on charcoal: 50% water) (9 g) under an atmosphere of hydrogen gas (3.5 bar) for (30h). The solution was filtered through kieselguhr and concentrated *in vacuo* to give 3-(methoxycarbonylmethyl)glutaric acid dimethylester as an oil, yield (84.9g, 94 %). NMR ¹H(CDCl₃), δ 2.48 (6H, d, J=8Hz, 3×CH₂), 2.78 (1H, hextet, J=δHz CH,) 3.7 (9H, s, 3×CH₃).
NMR ¹³C(CDCl₃), δ 28.6, CH; 37.50, 3×CH₃; 51.6, 3×CH₂; 172.28,3×COO.

### (Step c): Reduction and esterification of trimethyl ester to the triacetate.

Under an atmosphere of nitrogen in a 3 necked 2L round bottomed flask lithium aluminium hydride (20g, 588mmol) in tetrahydrofuran (400ml) was treated cautiously with *tris*(methyloxycarbonylmethyl)methane (40g, 212mmol) in tetrahydrofuran (200ml) over 1h. A strongly exothermic reaction occurred, causing the solvent to reflux strongly. The reaction was heated on an oil bath at 90°C at reflux for 3 days. The reaction was quenched by the cautious dropwise addition of acetic acid (100ml) until the evolution of hydrogen ceased. The stirred reaction mixture was cautiously treated with acetic anhydride solution (500ml) at such a rate as to cause gentle reflux. The flask was equipped for distillation and stirred and then heating at 90°C (oil bath temperature) to distil out the tetrahydrofuran. A further portion of acetic anhydride (300ml) was added, the reaction returned to reflux configuration and stirred and heated in an oil bath at 140°C for 5h. The reaction was allowed to cool and filtered. The aluminium oxide precipitate was washed with ethyl acetate and the combined filtrates concentrated on a rotary evaporator at a water bath temperature of 50°C *in vacuo* (5 mmHg) to afford an oil. The oil was taken up in ethyl acetate (500ml) and washed with saturated aqueous potassium carbonate solution The ethyl acetate solution was separated, dried over sodium sulphate, and concentrated *in vacuo* to afford an oil. The oil was Kugelrohr distilled in high vacuum to give *tris*(2-acetoxyethyl)methane (45.3g, 96%) as an oil. Bp. 220°C at 0.1 mmHg.
NMR¹H(CDCl₃), δ 1.66(7H,m, 3xCH₂, CH), 208(1H, s, 3xCH₃); 4.1(6H, t, 3×CH₂O). NMR ¹³C(CDCl₃), δ 20.9, CH₃; 29.34; CH; 32.17, CH₂; 62.15, CH₂O; 171, CO.

### (Step d): Removal of Acetate groups from the triacetate.

Tris(2-acetoxyethyl)methane (45.3g, 165mM) in methanol (200ml) and 880 ammonia (100ml) was heated on an oil bath at 80°C for 2 days. The reaction was treated with a further portion of 880 ammonia (50ml) and heated at 80°C in an oil bath for 24h. A further portion of 880 ammonia (50ml) was added and the reaction heated at 80°C for 24h. The reaction was then concentrated *in vacuo* to remove all solvents to give an oil. This was taken up into 880 ammonia (150ml) and heated at 80°C for 24h. The reaction was then concentrated *in vacuo* to remove all solvents to give an oil.
Kugelrohr distillation gave acetamide bp 170-180 0.2mm. The bulbs containing the acetamide were washed clean and the distillation continued. *Tris(2-*hydroxyethyl)methane (22.53g, 92%) distilled at bp 220 °C 0.2mm.
NMR ¹H(CDCl₃), δ 1.45(6H, q, 3xCH₂), 2.2(1H, quintet, CH); 3.7(6H, t 3xCH₂OH); 5.5(3H, brs, 3xOH).
NMR ¹³C(CDCl₃), δ 22.13, CH; 33.95, 3×CH₂; 57.8, 3×CH₂OH.

### (Step e): Conversion of the triol to the tris(methanesulphonate).

To an stirred ice-cooled solution of *tris*(2-hydroxyethyl)methane (10g, 0.0676mol) in dichloromethane (50ml) was slowly dripped a solution of methanesulphonyl chloride (40g, 0.349mol) in dichloromethane (50ml) under nitrogen at such a rate that the temperature did not rise above 15°C. Pyridine (21.4g, 0.27mol, 4eq) dissolved in dichloromethane (50ml) was then added drop-wise at such a rate that the temperature did not rise above 15°C, exothermic reaction. The reaction was left to stir at room temperature for 24h and then treated with 5N hydrochloric acid solution (80ml) and the layers separated. The aqueous layer was extracted with further dichloromethane (50ml) and the organic extracts combined, dried over sodium sulphate, filtered and concentrated *in vacuo* to give tris[2-(methylsulphonyloxy)ethyl]methane contaminated with excess methanesulphonyl chloride. The theoretical yield was 25.8g.
NMR ¹H(CDCl₃), δ 4.3 (6H, t, 2xCH₂), 3.0 (9H, s, 3×CH₃), 2(1H, hextet, CH), 1.85 (6H, q, 3×CH₂).

### (Step f): Preparation of 1,1,1-tris(2-azidoethyl)methane.

A stirred solution of *tris*[2-(methylsulphonyloxy)ethyl]methane [from Step 1(e), contaminated with excess methylsulphonyl chloride] (25.8g, 67mmol, theoretical) in dry DMF (250ml) under nitrogen was treated wtih sodium azide (30.7g, 0.47 mol) portion-wise over 15 minutes. An exotherm was observed and the reaction was cooled on an ice bath. After 30 minutes, the reaction mixture was heated on an oil bath at 50°C for 24h. The reaction became brown in colour. The reaction was allowed to cool, treated with dilute potassium carbonate solution (200ml) and extracted three times with 40/60 petrol ether/diethylether 10:1 (3×150ml). The organic extracts were washed with water (2×150ml), dried over sodium sulphate and filtered. Ethanol (200ml) was added to the petrol/ether solution to keep the triazide in solution and the volume reduced *in vacuo* to no less than 200ml. Ethanol (200ml) was added and reconcentrated *in vacuo* to remove the last traces of petrol leaving no less than 200ml of ethanolic solution. The ethanol solution of triazide was used directly in Step 1(g).
**CARE:** DO NOT REMOVE ALL THE SOLVENT AS THE AZIDE IS POTENTIALLY EXPLOSIVE AND SHOULD BE KEPT IN DILUTE SOLUTION AT ALL TIMES.
Less than 0.2ml of the solution was evaporated in vacuum to remove the ethanol and an NMR run on this small sample:
NMR ¹H(CDCl₃), δ 3.35 (6H, t, 3xCH₂), 1.8 (1H, septet, CH, ), 1.6 (6H, q, 3xCH₂).

### (Step g): Preparation of 1,1,1-tris(2-aminoethyl)methane.

*Tris*(2-azidoethyl)methane (15.06g, 0.0676 mol), (assuming 100% yield from previous reaction) in ethanol (200ml) was treated with 10% palladium on charcoal (2g, 50% water) and hydrogenated for 12h. The reaction vessel was evacuated every 2 hours to remove nitrogen evolved from the reaction and refilled with hydrogen. A sample was taken for NMR analysis to confirm complete conversion of the triazide to the triamine. **Caution: unreduced azide could explode on distillation.** The reaction was filtered through a Celite pad to remove the catalyst and concentrated *in vacuo* to give *tris(2-*aminoethyl)methane as an oil. This was further purified by Kugelrohr distillation bp.180-200°C at 0.4mm/Hg to give a colourless oil (8.1g, 82.7% overall yield from the triol).
NMR ¹H(CDCl₃), 272 (6H, t, 3×CH₂N), 1.41 (H, septet, CH), 139 (6H, q, 3×CH₂). NMR ¹³C(CDCl₃), δ39.8(CH₂NH₂), 38.2 (CH₂), 31.0(CH).

### Example 2: Alternative Preparation of 1,1,1-tris(2-aminoethyl)methane.

### (Step a): Amidation of trimethylester with p-methoxy-benzylamine.

*Tris*(methyloxycarbonylmethyl) methane [2g, 8.4 mmol; prepared as in Step 1(b). above] was dissolved in *p*-methoxy-benzylamine (25 g, 178.6 mmol). The apparatus was set up for distillation and heated to 120 °C for 24 hrs under nitrogen flow. The progress of the reaction was monitored by the amount of methanol collected. The reaction mixture was cooled to ambient temperature and 30 ml of ethyl acetate was added, then the precipitated triamide product stirred for 30 min. The triamide was isolated by filtration and the filter cake washed several times with sufficient amounts of ethyl acetate to remove excess *p*-methoxy-benzylamine. After drying 4.6 g, 100 %, of a white powder was obtained. The highly insoluble product was used directly in the next step without further purification or characterisation.

### (Step b): Preparation of 1,1,1-tris[2-(p-methoxybenzylamino)ethyl]methane.

To a 1000 ml 3-necked round bottomed flask cooled in a ice-water bath the triamide from step 2(a) (10 g, 17.89 mmol) is carefully added to 250 ml of 1M borane solution (3.5 g, 244.3 mmol) borane. After complete addition the ice-water bath is removed and the reaction mixture slowly heated to 60 °C. The reaction mixture is stirred at 60 °C for 20 hrs. A sample of the reaction mixture (1 ml) was withdrawn, and mixed with 0.5 ml 5N HCl and left standing for 30 min. To the sample 0.5 ml of 50 NaOH was added, followed by 2 ml of water and the solution was stirred until all of the white precipitate dissolved. The solution was extracted with ether (5 ml) and evaporated. The residue was dissolved in acetonitrile at a concentration of 1 mg/ml and analysed by MS. If mono- and diamide (M+H/z = 520 and 534) are seen in the MS spectrum, the reaction is not complete. To complete the reaction, a further 100 ml of 1M borane THF solution is added and the reaction mixture stirred for 6 more hrs at 60 °C and a new sample withdrawn following the previous sampling procedure. Further addition of the 1M borane in THF solution is continued as necessary until there is complete conversion to the triamine.

The reaction mixture is cooled to ambient temperature and 5N HCl is slowly added, [CARE: vigorous foam formation occurs!]. HCl was added until no more gas evolution is observed. The mixture was stirred for 30 min and then evaporated. The cake was suspended in aqueous NaOH solution (20-40 %; 1:2 w/v) and stirred for 30 minutes. The mixture was then diluted with water (3 volumes). The mixture was then extracted with diethylether (2 x 150 ml) [CARE: do not use halogenated solvents]. The combined organic phases were then washed with water (1×200 ml), brine (150 ml) and dried over magnesium sulphate Yield after evaporation 7.6 g, 84 % as oil.
NMR ¹H(CDCl₃), δ: 1.45, (6H, m, 3xCH₂; 1.54, (1H, septet, CH); 2.60 (6H, t, 3×CH₂N); 3.68 (6H, s, ArCH₂); 3.78 (9H, s, 3×CH₃O); 6.94(6H, d, 6xAr). 7.20(6H, d, 6xAr).
NMR ¹³C(CDCl₃), δ: 32.17,CH; 34.44, CH₂; 47.00, CH₂; 53.56, ArCH₂; 55.25, CH₃O; 113.78, Ar; 129.29, Ar; 132.61; Ar; 158.60, Ar;

### (Step c): Preparation of 1.1.1-tris(2-aminoethyl)methane.

1,1,1-*tris*[2-(*p*-methoxybenzylamino)ethyl]methane (20.0 gram, 0.036 mol) was dissolved in methanol (100 ml) and Pd(OH)₂ (5.0 gram) was added. The mixture was hydrogenated (3 bar, 100°C, in an autoclave) and stirred for 5 hours. Pd(OH)₂ was added in two more portions (2 x 5 gram) after 10 and 15 hours respectively. The reaction mixture was filtered and the filtrate was washed with methanol. The combined organic phase was evaporated and the residue was distilled under vacuum (1 x 10 ⁻², 110 °C) to give 2.60 gram (50 %) of 1,1,1-tris(2-aminoethyl)methane identical with the previously described Example 1.

### Example 3: Preparation of 3-chloro-3-methyl-2-nitrosobutane.

A mixture of 2-methylbut-2-ene (147ml, 1.4mol) and isoamyl nitrite (156ml, 1.16mol) was cooled to -30°C in a bath of cardice and methanol and vigorously stirred with an overhead air stirrer and treated dropwise with concentrated hydrochloric acid (140ml, 1.68mol ) at such a rate that the temperature was maintained below -20°C. This requires about 1h as there is a significant exotherm and care must be taken to prevent overheating. Ethanol (100ml) was added to reduce the viscosity of the slurry that had formed at the end of the addition and the reaction stirred at -20 to -10°C for a further 2h to complete the reaction. The precipitate was collected by filtration under vacuum and washed with 4×30ml of cold (-20°C) ethanol and 100ml of ice cold water, and dried *in vacuo* to give 3-chloro-3-methyl-2-nitrosobutane as a white solid. The ethanol filtrate and washings were combined and diluted with water (200ml) and cooled and allowed to stand for 1h at -10°C when a further crop of 3-chloro-3-methy-2-nitrosobutane crystallised out. The precipitate was collected by filtration and washed with the minimum of water and dried *in vacuo* to give a total yield of 3-chloro-3-methyl-2-nitrosobutane (115g 0.85mol, 73%) >98% pure by NMR.
NMR ¹H(CDCl₃), As a mixture of isomers (isomer1, 90%) 1.5 d, (2H, CH₃), 1.65 d, (4H, 2 xCH₃), 5.85,q, and 5.95,q, together 1H (isomer2, 10%), 1.76 s (6H, 2x CH₃), 2.07(3H, CH₃).

### Example 4: Synthesis of bis [N-(1,1-dimethyl-2-N-hydroxyimine propyl)2-aminoethyl]-(2-aminoethyl)methane (Chelator 1).

To a solution of *tris*(2-aminoethyl)methane (4.047g, 27.9mmol) in dry ethanol (30ml) was added potassium carbonate anhydrous (7.7g, 55.8mmol, 2eq) at room temperature with vigorous stirring under a nitrogen atmosphere. A solution of 3-chloro-3-methyl-2-nitrosobutane (7.56g, 55.8mol, 2eq) was dissolved in dry ethanol (100ml) and 75ml of this solution was dripped slowly into the reaction mixture. The reaction was followed by TLC on silica [plates run in dichloromethane, methanol, concentrated (0.88sg) ammonia; 100/30/5 and the TLC plate developed by spraying with ninhydrin and heating]. The mono-, di- and tri-alkylated products were seen with RF's increasing in that order. Analytical HPLC was run using RPR reverse phase column in a gradient of 7.5-75% acetonitrile in 3% aqueous ammonia. The reaction was concentrated *in vacuo* to remove the ethanol and resuspended in water (110ml). The aqueous slurry was extracted with ether (100ml) to remove some of the trialkylated compound and lipophilic impurities leaving the mono and desired dialkylated product in the water layer. The aqueous solution was buffered with ammonium acetate (2eq, 4.3g, 55.8mmol) to ensure good chromatography. The aqueous solution was stored at 4°C overnight before purifying by automated preparative HPLC.
Yield (2.2g, 6.4mmol, 23%).
Mass spec; Positive ion 10 V cone voltage. Found: 344; calculated M+H= 344.
NMR ¹H(CDCl₃), δ 1.24(6H, s, 2xCH₃), 1.3(6H, s, 2xCH₃), 1.25-1.75(7H, m, 3xCH₂,CH), (3H, s, 2xCH₂), 2.58 (4H, m, CH₂N), 2.88(2H, t CH₂N₂), 5.0 (6H, s, NH₂ , 2xNH, 2xOH).
NMR ¹H ((CD₃)₂SO) δ1.1 4xCH; 1.29, 3xCH₂; 2.1 (4H, t, 2xCH₂);
NMR ¹³C((CD₃)₂SO), δ 9.0 (4xCH₃), 25.8 (2xCH₃), 31.0 2xCH₂, 34.6 CH₂, 56.8 2xCH₂N; 160.3, C=N.

HPLC conditions: flow rate 8ml/min using a 25mm PRP column A=3% ammonia solution (sp.gr = 0.88) /water; B = Acetonitrile

| Time | %B |
|---|---|
| 0 | 7.5 |
| 15 | 75.0 |
| 20 | 75.0 |
| 22 | 7.5 |
| 30 | 7.5 |

Load 3ml of aqueous solution per run, and collect in a time window of 12.5-13.5 min.

### Example 5: Synthesis of 3-1(4'-Fluorobiphenyl-4-sulfonyl)-(I-hydroxycarbamoylcyclopentyl)amino]propionic Acid (Compound 27; Prior Art).

(Step A) To a solution of 1-aminocyclopentanecarboxylic acid benzyl ester p-toluenesulfonic acid salt (12.1 grams, 30.9 mmol) and triethylamine (10.0 mL, 72 mmol) in water (150 mL) and 1,4-dioxane (150 mL) was added 4'- fluorobiphenyl-4-sulfonyl chloride (8.8 grams, 32.5 mmol). The mixture was stirred at room temperature for 16 hours and then most of the solvent was removed by evaporation under vacuum. The mixture was diluted with ethyl acetate and was washed successively with dilute hydrochloric acid solution, water, and brine. The solution was dried over magnesium sulfate and concentrated to leave 1-(4'-fluorobiphenyl-4-sulfonylamino) cyclopentanecarboxylic acid benzyl ester as a solid, 12.33 grams (76%).

(Step B) To a solution of 1-(4'-fluorobiphenyl-4-sulfonylamino) cyclopentanecarboxylic acid benzyl ester (23.0 grams, 50.7 mmol) in dry DMF (500 ml) at room temperature was added potassium hexamethyldisilazide (12.2 grams, 61.1 mmole) and, after 45 minutes, tert- butyl-(3-iodopropoxy)dimethylsilane (18.3 grams, 60.9 mmol). The resulting mixture was stirred at room temperature for 16 hours. Additional potassium hexamethyldisilazide (3.0 grams, 15 mmole) and tert- butyl-(3-iodopropoxy)-dimethylsilane (4.5 grams, 15 mmol) were then added. Stirring at room temperature was continued for a further 5 hours. The mixture was quenched by addition of saturated ammonium chloride solution. The DMF was removed by evaporation under vacuum. The residue was taken up in diethyl ether and washed successively with water, dilute aqueous hydrochloric acid solution and brine. After drying over magnesium sulfate, the diethyl ether was evaporated to afford a yellow oil. To this was added hexane and methylene chloride to induce crystallization of the starting material which was recovered by filtration. Evaporation of solvents from the filtrate afforded crude 1-[[3-(tert-butyl-dimethylsilanyloxy)propyl)-(4'-fluorobiphenyl-4-sulfonyl) amino]-cyclopentanecacboxylic acid benzyl ester as an amber oil (27.35 grams).

(Step C) To a solution of the crude 1-[[3-(tert-butyl-dimethylsilanyloxy)propyl]-(4'-fluorobiphenyl-4-sulfonyl)amino]cyclopentanecarboxylic acid benzyl ester (27.35 grams) in methylene chloride (450 mL) at room temperature was added boron trifluoride etherate (11 mL, 89.4 mmol). After 45 minutes, the reaction was quenched by sequential addition of saturated ammonium chloride solution and water. The organic phase was separated, washed with water and brine and dried over magnesium sulphate. Evaporation of the solvent under vacuum provided crude 1-[(4'-fluorobiphenyl-4-sulfonyl)-(3-hydroxypropyl)amino]-cyclopentane carboxylic acid benzyl ester as an amber oil (22.1 grams).

(Step D) A solution of the crude 1-[(4'-fluorobiphenyl-4-sulfonyl)-(3-hydroxypropyl)amino]-cyclopentanecarboxylic acid benzyl ester (22.1 grams) in acetone (400 mL) was cooled in an ice bath and treated with Jones reagent (about 20 mL) until an orange colour persisted. The mixture was stirred from 0°C to room temperature over 2 hours. After quenching excess oxidant with isopropanol (1 mL), Celite® was added and the mixture was filtered. The filtrate was concentrated under vacuum. The residue was taken up in ethyl acetate, washed with water and brine, dried over magnesium sulphate and concentrated to afford crude 1-[(2-carboxyethyl)-(4'-fluorobiphenyl-4-sulfonyl)amino]-cyclopentanecarboxylic acid benzyl ester as an oil (21.4 grams).

(Step E) To a solution of the crude 1-[(2-carboxyethyl)-(4'-fluorobiphenyl-4-sulfonyl)amino]- cyclopentanecarboxylic acid benzyl ester (21.4 grams) in DMF (500 mL) at room temperature was added potassium carbonate (22.5 grams, 163 mmol) and methyl iodide (3.7 mL, 59.4 mmol). The mixture was stirred for 16 hours at room temperature and was then concentrated under vacuum. The residue was taken up in water and acidified using 6N aqueous hydrogen chloride solution. The resulting mixture was extracted with a mixture of diethyl ether and ethyl acetate. The organic extract was washed with water and brine, dried over magnesium sulphate. After concentration to an amber oil, 1-[(4'-fluorobiphenyl-4-sulfonyl)-(2-methoxycarbonylethyl)amino]-cyclopentane-1-carboxylic acid benzyl ester (12.6 grams), a white solid, was isolated by flash chromatography on silica gel eluting with 15% ethyl acetate in hexane.

(Step F) A solution of 1-[(4'-fluorobiphenyl-4-sulfonyl)-(2-metboxycarbonylethyl) amino]-cyclopentane-1-carboxylic acid benzyl ester (12.1 grams, 22.4 mmole) in methanol (270 mL) was treated with 10% palladium on activated carbon and hydrogenated in a Parr® shaker at 3 atmospheres pressure for 3.5 hours. After filtration through nylon (pore size 0.45 µm) to remove the catalyst, the solvent was evaporated to afford 1-{(4'-fluorobiphenyl-4-sulfonyl)-(2-methoxycarbonylethyl)amino]cyclopentane-1-carboxylic acid as a white foam (10.1 grams, 100%).

(Step G) Diisopropylethylamine (4.3 mL, 24.6 mmol) and (benzotriazol-1-yloxy)tris-(dimethylamino)phosphonium hexafluorophosphate (11.0 grams, 24. 9 mmol) were added sequentially to a solution of 1-[(4'-fluorobiphenyl-4-sulfonyl)-(2-methoxycarbonylethyl)-amino]cyclopentane-1-carboxylic acid (10.1 grams, 22.4 mmole) in N,N-dimethylformamide (170 mL). The mixture was stirred for 4 hours. Additional diisopropylethylamine (7.8 mL, 44.6 mmol) and O-benzylhydroxylamine hydrochloride (4.64 grams, 29.1 mmol) were then added and the resulting mixture was stirred at 60°C for 16 hours. After concentration under vacuum, the residue was taken up in water and acidified with 1N aqueous hydrogen chloride solution. The mixture was extracted with ethyl acetate and the extract was washed sequentially with water, saturated aqueous sodium bicarbonate solution and brine. The solution was dried over magnesium sulphate and concentrated to give a solid which upon trituration with 7:3:1 hexane/ethyl acetate/ methylene chloride provided 3-[(1-benzyloxycarbamoylcyclopentyl)- (4'- fluorobiphenyl-4-sulfonyl)amino]propionic acid methyl ester as a white crystalline solid (10.65 grams, 86%).

(Step H) A solution of 3-[(1-benzyloxycarbamoylcyclopentyl)-(4'-fluorobiphenyl-4-sulfonyl)amino]propionic acid methyl ester (10.65 grams, 19.2 mmol) in methanol (250 mL) was treated with 5% palladium on barium sulphate and hydrogenated in a Parr® shaker at 3 atmospheres pressure for 3 hours. After filtration through nylon (pore size 0.45 µm) to remove the catalyst, the solvent was evaporated to afford 3-[(4'-fluorobiphenyl-4-sulfonyl)-(1-hydroxycarbamoylcydopentyl)amino)propionic acid methyl ester as a white foam (8.9 grams, 100%).
¹H NMR (DMSO-d₆) δ 8.80 (br s, 1H), 7.85-7.75 (m, 6 H), 732-725 (m, 2.H), 354 (s, 3 H), 3.52-3.48 (m, 2 H), 2.73-2.69 (m, 2 H), 2.24-2.21 (m, 2 H), 1.86-1. 83 (m, 2 H), 1.60-1.40 (m, 4 H).

(Step I) A solution of 3-[(4'-fluorobiphenyl-4-sulfonyl)-(1-hydroxycarbamoylcyclopentyl)amino]-propionic add methyl ester (8.9 grams, 19.2. mmol) in methanol (500 mL) was treated with aqueous 1N sodium hydroxide solution (95 mL, 95 mmol) and stirred at room temperature for 5.5 hours. The mixture was concentrated to remove methanol, diluted with water, acidified with 6 N aqueous hydrochloric acid solution and extracted with ethyl acetate. After washing with water and brine the organic extract was dried over magnesium sulphate and concentrated to afford 3-[(4'-fluorobiphenyl-4-sulfonyl)-(1-hydroxycarbamoyl-, cyclopentyl)amino]propionic acid as a white foam which was crystallised from ethyl acetate (6.74 grams, 78%). Mp: 163-164°C.
¹ H NMR (DMSO-d₆) δ 12.30 (br s, 1H), 10.40 (br s, 1H), 8.77 (br s, 1H), 7.89-7.74 (m, 6H), 7.31-7.27 (m, 2H), 3.51-3.44 (m, 2H), 2.64-2.60 (m, 2H), 2.24-2.22 (m, 2H), 1.86-1.83 (m, 2H), 1.60-1.40 (m, 4H). MS 449 (M-1).

### Example 6: Synthesis of Compound 23.

To a stirred solution of Compound 1, O-(1H-Benzotriazol-1-yl)-N, N, N', N'-tetramethyluronium tetrafluoroborate or TBTU and N-methylmorpholine in DMF was added tyramine. The reaction mixture was allowed to react for 24 hours at room temperature under inert atmosphere. The reaction was monitored *via* HPLC. After completion the yellow clear solution was concentrated and dried under high vacuum for four hours. The crude product was purified *via* HPLC preparative and yielded 88% of an off white solid.
¹H NMR (DMSO): δ10.5 (1H, s, NH);9.3 (1H, s, NH);'8.8 (1H, s, OH);8 (1H, s, OH);'7.8 (2H, J = 8.8 Hz, d,Har); 7.3 (2H, J= 8Hz, t, Har); 7.2 (2H, d, J= 8Hz, Har); 7.1 (2H, J=8.8 Hz, d, Har); 7 (2H, J= 8.8 Hz, d, Har); 6.7 (2H, J= 8.8 Hz, d, 2H); 3.4 (2H, m, CH₂); 3.1 (2H, m, CH₂); 2.7 (2H, m, CH₂), 2.6 (2H, m, CH₂); 2.2-1.9 (4H, m, CH₂); 1.5 (4H, m, CH₂)
MS: (ESI) 586.2 (MH⁺) and 608.2 (MNa⁺)
HPLC: 98% pure.

### Example 7: Synthesis of Compound 26.

To a stirred solution of Compound 1, (7-Azabenzotriazole-1-yloxy)tripyrrolidinophosphonium hexafluorophosphate (PyAOP) and N-methylmorpholine in DMF was added iodoaniline. The reaction mixture was allowed to react for 3 days at room temperature under inert atmosphere. The reaction was monitored by HPLC. After completion the solution was concentrated and dried under high vacuum for four hours. The crude product was purified by preparative HPLC and yielded 21 % of solid.
MS: (ESI) 668 (MH⁺) and 690 (MNa⁺)
HPLC: 100% pure.

### Example 8: Synthesis of Compound 24.

### Step A: Preparation of 3-iodotyramine.

Iodine solution (1M, 2ml) was added slowly at room temperature to 20 ml of a tyramine solution (50mM in 30% ammonia). After 5 hours the solution was concentrated to 5 ml and left overnight at 0°C. The off-white precipitate formed was filtrated and washed with cold water. The solid was dried under high vacuum overnight.
¹H NMR (CD₃OD): δ 2.7 (2H, t, J= 7Hz); 2.9 (2H, t, J=7Hz); 6.7 (1H, d, J=8.1Hz); 7.1 (1H, dd, J= 2.2Hz, 8Hz); 7.5 (1H, d, J=2.2Hz).

### Step B.

To a stirred solution of Compound 1, O-(1H-Benzotriazol-1-yl)-N, N, N', N'-tetramethyluronium tetrafluoroborate (TBTU) and N-methylmorpholine in DMF was added 3-iodotyramine (from Step A). The reaction mixture was allowed to react for 24 hours at room temperature under inert atmosphere. The reaction was monitored *via* HPLC. After completion the yellow clear solution was concentrated and dried under high vacuum for four hours. The crude product was purified by preparative HPLC and yielded 10% of an off white solid (Compound 24).
MS (ESI): 712 (MH⁺) 734 (MNa⁺)

### Example 9: Synthesis of A Chelator-MMPi Conjugate (Compound 2).

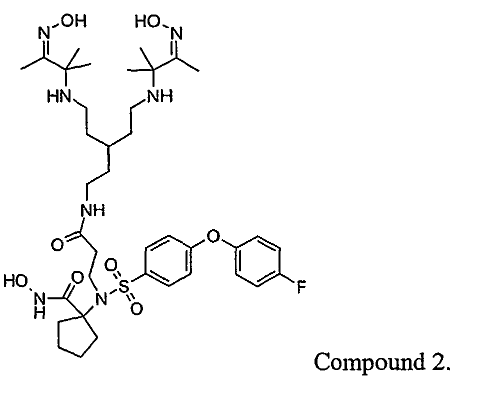

Compound 1 (5.1 mg), PyAOP (6.0 mg) and N-methylmorpholine (2 µL) were dissolved in dimethylformamide (0.5 mL) and the mixture stirred for 2 minutes. Chelator 1 (3.4 mg) was added and the reaction mixture stirred overnight. 20 % acetonitrile/water (8 mL) was added and the product purified using preparative HPLC (column: Phenomenex Luna 10µ C18 (2) 250 x 10 mm, detection: 230 nm, solvent A: H₂O/0.1 % TFA, solvent B: CH₃CN/0.1 % TFA, flow: 5 mL/min, gradient: 20-60 % B over 30 min, t_{R}: 17 min). After lyophilisation 1 mg pure material was obtained and characterised by LC-MS (column: Phenomenex Luna 5µ C18 (2) 250 x 4.6 mm, detection: 214 nm, solvent A: H₂O/0.1 % TFA, solvent B: CH₃CN/0.1 % TFA, flow: 1 mL/min, gradient: 20-60 % B over 20 min, t_{R}: 14.32 min, found m/z: 792.5, expected MH⁺: 792.4).

### Example 10:Synthesis of an Amino-PEG linker derivatised MMPi (Compound 4).

### Step (a) 1,11 -Diazido-3,6,9-trioxaundecane.

A solution of dry tetraethylene glycol (19.4 g, 0.100 mol) and methanesulphonyl chloride (25.2 g, 0.220 mol) in dry THF (100 ml) was kept under argon and cooled to 0 °C in an ice/water bath. To the flask was added a solution of triethylamine (22.6 g, 0.220 mol) in dry THF (25 ml) dropwise over 45 min. After I hr the cooling bath was removed and stirring was continued for 4 hrs. Water (60 ml) was added. To the mixture was added sodium hydrogen carbonate (6g, to pH 8) and sodium azide (14.3 g, 0.220 mmol), in that order. THP was removed by distillation and the aqueous solution was refluxed for 24 h (two layers formed). The mixture was cooled and ether (100 ml) was added. The aqueous phase was saturated with sodium chloride. The phases were separated and the aqueous phase was extracted with ether (4 x 50 ml). Combined organic phases were washed with brine (2 x 50 ml) and dried (MgSO₄). Filtration and concentration gave 22.1 g (91 %) of yellow oil. The product was used in the next step without further purification.

### Step (b) 11-Azido-3,6,9-trioxaundecanamine.

To a mechanically, vigorously stirred suspension of 1,11-diazido-3,6,9-trioxaundecane (20.8 g, 0.085 mol) in 5% hydrochloric acid (200 ml) was added a solution of triphenylphosphine (19.9 g, 0.073 mol) in ether (150 ml) over 3 hrs at room temperature. The reaction mixture was stirred for additional 24 hrs. The phases were separated and the aqueous phase was extracted with dichloromethane (3 x 40 ml). The aqueous phase was cooled in an ice/water bath and pH was adjusted to ca 12 by addition of KOH. The product was extracted into dichloromethane (5 x 50 ml). Combined organic phases were dried (MgSO₄). Filtration and evaporation gave 14.0 g (88%) of yellow oil. Analysis by MALDI-TOF mass spectroscopy (matrix: □-cyano-4-hydroxycinnamic acid) gave a M+H peak at 219 as expected. Further characterisation using ¹H (500 MHz) and ¹³C (125 MHz) NMR spectroscopy verified the structure.

### Step (c) Synthesis of (Compound 1)-PEG(3)-N₃.

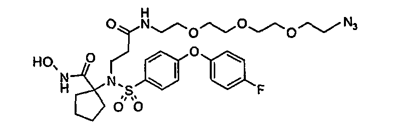

To a solution of Compound 1 (41 mg, 87 µmol) in DMF (5 ml) were added 11-azido-3,6,9-trioxaundecanamine (19 mg, 87 µmol), HATU (Applied.Biosystems, 33 mg, 87 µmol) and DIEA (Fluka, 30 µl, 174 µmol). After one hour reaction time the mixture was concentrated and the residue was purified by preparative HPLC (column Phenomenex Luna C18(2) 5 µm 21.2 x 250 mm, solvents: A = water/0.1% TFA and B = acetonitrile/0.1% TFA; gradient 30-60% B over 60 min; flow 10.0 ml/min, UV detection at 214 nm), giving 33.9mg(59%)of product after lyophilisation LC-MS analysis (column Phenomenex Luna C18(2) 3 µm 50 x 4.60 mm, solvents: A = water/0,1% TEA and B = acetonitrile/0.1 % TFA; gradient 20-100% B over 10 min; flow 1 ml/min, UV detection at 214 nm, ESI-MS) gave a peak at 4.88 min with m/z 667.4 (MH⁺) as expected.

### Step (d) Synthesis of Compound 4.

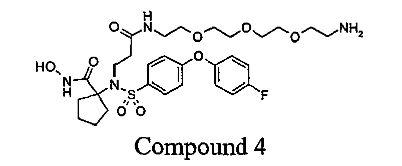

To a solution of (Compound 1)-PEG(3)-N₃ (4.7 mg, 7 µmol) in methanol (4 ml) was added Pd/C (Koch-Light, ca 10 mg) added. The mixture was stirred at room temperature under hydrogen atmosphere (1 atm) for 10 min. The mixture was filtered and concentrated. LC-MS analysis (column Phenomenex Luna C18(2) 3 µm 50 x 4.60 mm, solvents: A = water/0.1 % TFA and B = acetonitrile/0. 1% TFA; gradient 20-100% B over 10 min; flow 1 ml/min, UV detection at 214 nm, ESI-MS) gave a peak at 4.17 min with m/z 641.4 (MH⁺) as expected. The product was used directly in subsequent steps without further purification.

### Example 11 : Synthesis of Chelator Conjugate with PEG(3)-Diglycolyl Spacer (Compound 3).

### Step (a) Synthesis of (Compound 1)-PEG(3)-Diglycolic acid.

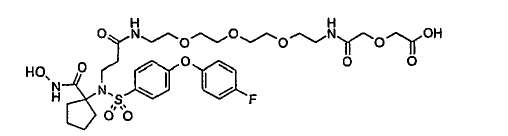

To a solution of (Compound 1)-PEG(3)-NH₂ (Example 6, 25 mg, 39 µmol) in DMF (4 ml) was added diglycolic anhydride (Acres, 9 mg, 78 µmol). After stirring for 1.5 hrs the reaction mixture was concentrated and the residue purified by preparative HPLC (column Phenomenex Luna C18(2) 5 µm 212 x 250 mm, solvents: A=wate/0.1% TFA and B = acetonitrile/0.1% TFA; gradient 20-80% B over 60 mm; flow 10.0 ml/min, UV detection at 214 nm), giving 14.9 mg (51%) of lyophilised material. The product was analysed by LC-MS (column Phenomenex Luna C18(2) 3 µm 50 x 4.60 mm, solvents: A=water/0.1%.TFA and B=acetonitrile/0.1% TFA; gradient 20-100% B over 10 min; flow 1 ml/min, UV detection at 214 nm, EST-MS,) giving a peak at 4.15 min with m/z 757.3 (MH⁺) corresponding to the product. Further characterisation was carried out using NMR spectroscopy.

### Step (b): Synthesis of Compound 3.

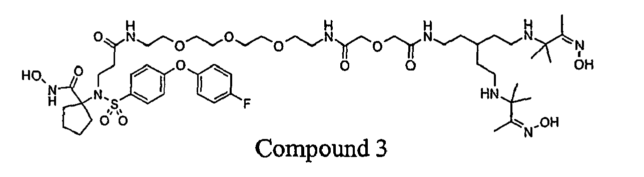

To a solution of (Compound 1)-PEG(3)-Diglycolic acid (6.6 mg, 9 µmol) in DMF (3 ml) were added Chelator 1 (3.1 mg, 9 µmol), HATU (Applied Biosystems, 3.4 mg, 9 µmol) and DIEA (Fluka, 3.1 µl, 18 µmol). After 20 min the reaction time the mixture was concentrated and the residue was purified by preparative HPLC (column Phenomenex Luna C18(2) 5 µm 21.2 x 250 mm, solvents: A = water/0.1% TFA and B = acetonitrile/0.1% TFA; gradient 10-80 % B over 60 min; flow 10.0 ml/min, UV detection at 214 nm), giving 4.2 mg (43%) of lyophilised product. LC-MS analysis (column Phenomenex Luna C18(2) 3 µm 50 x 4.60 nun, solvents: A = water/0.1% TFA and B = acetonitrile/0.1% TFA; gradient 20-100% B over 10 min; flow 1 ml/min, UV detection at 214 nm, ESI-MS; t_{R} = 4.17 min, m/z 1082.5 (MH+)) and NMR spectroscopy confirmed the structure.

### Example 12: Synthesis of chloroacetyl derivative for PET imaging (Compound 5).

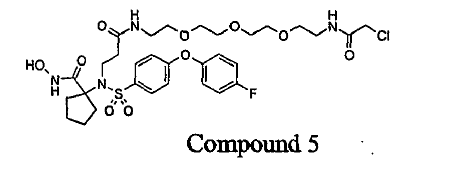

Freshly prepared chloroacetic anhydride (52 mg, 0.30 mmol) and DIEA (51 µl, 0.30 mmol) were added to a solution of Compound 4 (Example 10 step d, ca 0.15 mmol) in DMF (10 ml). After 1 hr the reaction mixture was concentrated and the residue was purified by preparative HPLC (column Phenomenex Luna C18(2) 10 µm 50 x 250 mm, solvents: A =water/0.1% TFA and B = acetomitrile/0.1% TFA; gradient 30-40% B over 60 min; flow 50.0ml/min, UV detection at 214nm), giving 25.8 mg (24%) of product after lyophilisation.. LC-MS analysis (column Phenomenex Luna C18(2) 3 µm 50 x 4.60 mm, solvents: A = water/0.1% TFA and B = acetonitrile/0.1% TFA; gradient 20-100% B over 10 min; flow 1 ml/min, UV detection at 214 nm, ESI-MS) gave a peak at 6.01 min with m/z 717.5 (MH⁺) as expected.

### Example 13: Conjugation of 3-fluoropropylthiol to chloroacetylated compound (Compound 6).

### Step (a) Synthesis of 3-tritylsulfanyl-propan-1-ol [Ph₃C-S(CH₂)₃OH].

Triphenylmethanol (390.6 mg, 1.5 mmol) in TFA (10 ml) was added dropwise to a stirred solution of 3-mercaptopropyl alcohol (129.6 µl, 1.5 mmol) in TFA (10 ml). After the addition TFA was evaporated under reduced pressure and the crude product immediately purified by reverse phase preparative chromatography (Phenomenex Luna C18 column, 00G-4253-V0; solvents A= water / 0.1% TFA and B= CH₃CN / 0.1 % TFA; gradient 70-80 % B over 60 min; flow 50 ml / minute; detection at 254 nm), affording 372 mg (74%) of pure compound. (analytical HPLC: Vydac C18 column, 218TP54: solvents: A= water / 0.1 % TFA and B= CH₃CN / 0.1 % TFA; gradient 70-80 % B over 20 min; flow 1.0 ml /minute; retention time 5.4 minutes detected at 214 and 254 nm). The structure was verified by NMR spectroscopy

### Step (b) Synthesis of methanesulfonic acid 3-tritylsulfanyl-propyl ester [Ph₃C-S(CH₂)₃O-Ms].

To a solution of 3-tritylsulfanyl-propan-1-ol (372.0 mg, 1.11 mmol) in THF (10 ml) was added triethylamine (151.7 mg, 209 µl, 1.5 mmol) and mesyl chloride (171.9 mg, 116.6 µl, 1.5 mmol). After 1 hour reaction time the precipitate was removed by filtration. The solution was concentrated and the residue was purified by reverse phase HPLC (Phenomenex Luna C18 column, 00G-4253-V0; solvents A= water / 0.1% TFA and B= CH₃CN/0.1% TFA; gradient 80-100 % B over 60 min; flow 50 ml / minute; detection at 254 nm), affording **318** mg (69%) of pure compound. (analytical HPLC: Vydac C18 column, 218TP54: solvents: A=water /0.1% TEA and B= CH₃CN / 0.1% TFA; gradient 60-70 % B over 20 min; flow 1.0 ml /minute; retention time 18.7 minutes detected at 214and 254 nm). The structure was verified by NMR spectroscopy.

### Step (c) Synthesis of (3- fluoro-propylsulfanyl)triphenylmethane [Ph₃C-S(CH₂)₃F].

Potassium fluoride (1.4 mg, 0.024 mmol) and Kryptofix 222 (9.0 mg, 0.024 mmol) were dissolved in acetonitrile (0.2 ml) (heating). A solution of methanesulfonic acid 3-tritylsulfanyl-propyl ester (5 mg, 0.012 mmol) in acetonitrile (0.2 ml) was added. The reaction mixture was heated at 80 °C for 90 min. The crude product was purified by reverse phase preparative chromatography (Vydac C18 column, 218TP1022; solvents A= water / 0.1% TFA and B= CH₃CN / 0.1% TFA; gradient 40-90 % B over 40 min; flow 10 ml / minute; detection at 254 nm). A yield of 2 mg (50%) of purified material was obtained (analytical HPLC: Phenomenex Luna C18 column, 00B-4251-E0: solvents: A= water / 0.1% TFA and B= CH₃CN / 0.1% TFA; gradient 40-80 % B over 10 min; flow 2.0 ml /minute; retention time 8.2 minutes detected at 214 and 254 nm). The structure was confirmed by NMR analysis.

### Step (d) Synthesis of Compound 6.

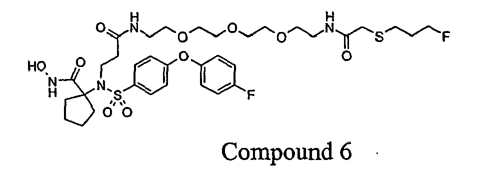

3-Fluoro-tritylsulfanyl-propane (1.4 mg, 4 µmol) was stirred in a mixture of TFA (50 µl), triisopropylsilane (5 µl) and water (5 µl). The mixture was added to a solution of Compound 5 (1.5 mg, 2 µmol) in 1:1 mixture of water and acetonitrile (800 µl). pH was adjusted to 10 by adding aqueous K₂CO₃ (200 µl, 0.5 g/ml) and the mixture was heated at 60°C for 25 min. The product was purified by preparative HPLC (column Phenomenex Luna C18(2) 5 µm 10.0 x 250 mm, solvents: A = water/0.1% TFA and B = acetonitrile/0.1% TFA; gradient 30-50% B over 60 min; flow 5.0 ml/min, UV detection at 214 nm), giving 0.9 mg (58%) of product. LC-MS analysis (column Phenomenex Luna C18(2) 3 µm 50 x 4.60 mm, solvents: A = water/0.1% TFA and B =acetonitrile/0.1% TFA; gradient 10-80% B over 10 min; flow 1 ml/min, UV detection at 214 nm, ESI-MS; t_{R} =6.25 min, m/z 775.4 (MH⁺)) confirmed the structure.

### Example 14 Chloroacetylated, Amino Acid and PEG derivatives for PET imaging (Compounds 7 to 22).

### [Compound 1]-Lys-Peg(4)-Diglycolyl-Lys(chloroacetyl)-NH₂ (Compound 7).

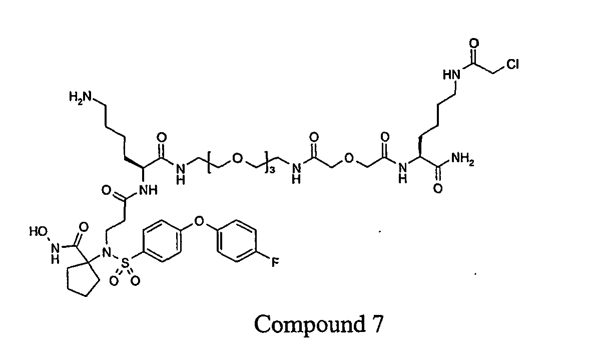

Compound 7 was synthesised using a manual nitrogen bubbler apparatus on a 0.05 mmol scale using Fmoc-protected Rink Amide MBHA resin (Novabiochem), Fmoc-Lys(Dde)-OH (Novabiochem), Fmoc-Lys(Boc)-OH (Novabiochem), Fmoc-amino-PEG-diglycolic acid (Polypure AS) and CP-471358 (Pfizer). All amino acids and CP-471358 were coupled using HATU/DIEA as coupling reagents. Reaction steps were analysed by Kaiser test. After coupling of the CP-compound side chain Dde group of C-terminal lysine was cleaved by standard hydrazine treatment. Chloroacetic acid (Fluka) was coupled by freshly prepared symmetrical anhydride. The simultaneous removal of product from the resin and cleavage of side-chain Boc protecting group was carried out in TFA containing 2.5% H₂O and 2.5% triisopropylsilane for 2 hours. Crude material was precipitated from ether and purified by preparative HPLC (column Phenomenex Luna C18(2) 10 µm 250 x 10 mm; solvents A = water/0.1 % TFA and B = acetonitrile/0.1 % TFA; gradient 20-40% B over 60 min; flow 5.0 ml/min; UV detection at 214 nm) to give 5.0 mg of white solid.
Analysis by LC-MS (column Phenomenex Luna C18(2) 3 µm 2.0 x 50 mm, solvents: A = water/0.1% TFA and B = acetonitrile/0.1% TFA; gradient 10-80% B over 10 min; flow 0.3 ml/min, UV detection at 214 and 254 nm, ESI-MS positive mode) gave a peak at 5.9 min with m/z 1088 as expected for MH+

Using solid phase peptide synthesis methodology, Compounds 8 to 22 were prepared in the same manner and characterised by MS.

### Example 15: Synthesis of the ¹⁸F-Labelled Derivative for N-alkylation:

### Synthesis of 3-[¹⁸F] fluoropropyl tosylate.

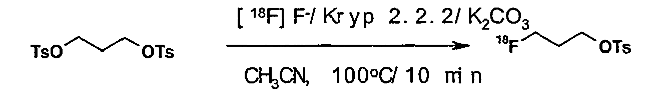

*Via* a two-way tap Kryptofix 222 (10mg) in acetonitrile (300 µl) and potassium carbonate (4mg) in water (300 µl), prepared in a glass vial, was transferred using a plastic syringe (1ml) into a carbon glass reaction vessel sited in a brass heater. ¹⁸F-fluoride (185-370MBq) in the target water (0.5-2ml) was then added through the two-way tap. The heater was set at 125°C and the timer started. After 15mins three aliquots of acetonitrile (0.5ml) were added at 1min intervals. The ¹⁸F-fluoride was dried up to 40mins in total. After 40mins, the heater was cooled down with compressed air, the pot lid was removed and 1,3-propanediol-di-p-tosylate (5-12mg) and acetonitrile (1ml) was added. The pot lid was replaced and the lines capped off with stoppers. The heater was set at 100°C and labelled at 100°C/10mins. After labelling, 3-[¹⁸F] fluoropropyl tosylate was isolated by Gilson RP HPLC using the following conditions:

| | |
|---|---|
| Column | u-bondapak C18 7.8x300mm |
| Eluent | Water (pump A): Acetonitrile (pump B) |
| Loop Size | 1ml |
| Pump speed | 4ml/min |
| Wavelength | 254nm |
| Gradient | 5-90% eluent B over 20 min |
| Product Rt | 12 min |

Once isolated, the cut sample *(ca.* 10ml) was diluted with water (10ml) and loaded onto a conditioned C18 sep pak. The sep pak was dried with nitrogen for 15mins and flushed off with an organic solvent, pyridine (2ml), acetonitrile (2ml) or DMF (2ml). Approx. 99% of the activity was flushed off.

- 3-[¹⁸F] fluoropropyl tosylate is used to N-alkylate amines by refluxing in pyridine.

### Example 16: [¹⁸F]-Thiol Derivative for S-alkylation.

### Step (a): Preparation of 3-[¹⁸F] fluoro-tritylsulfanyl-propane.

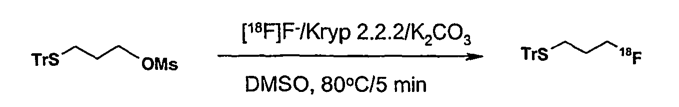

*Via* a two-way tap Kryptofix 222 (10mg) in acetonitrile (800 µl) and potassium carbonate (1mg) in water (50 µl), prepared in a glass vial, was transferred using a plastic syringe (1ml) to the carbon glass reaction vessel situated in the brass heater. ¹⁸F-fluoride (185-370 MBq) in the target water (0.5-2ml) was then also added through the two-way tap. The heater was set at 125°C and the timer started. After 15mins three aliquots of acetonitrile (0.5ml) were added at 1min intervals. The ¹⁸F-fluoride was dried up to 40mins in total. After 40mins, the heater was cooled down with compressed air, the pot lid was removed and trimethyl-(3-tritylsulfanylpropoxy)silane (1-2mg) and DMSO (0.2ml) was added. The pot lid was replaced and the lines capped off with stoppers. The heater was set at 80 °C and labelled at 80 °C/5mins. After labelling, the reaction mixture was analysed by RP HPLC using the following HPLC conditions:

| | | |
|---|---|---|
| Column | u-bondapak C18 7.8x300mm | |
| Eluent | 0.1 %TFA/Water (pump A): 0.1 %TFA/Acetonitrile (pump B) | |
| Loop Size | 100u1 | |
| Pump speed | 4ml/min | |
| Wavelength | 254nm | |
| Gradient | 1 mins 15 mins 5 mins | 40%B 40-80%B 80%B |

The reaction mixture was diluted with DMSO/water (1:1 v/v, 0.15ml) and loaded onto a conditioned t-C18 sep-pak. The cartridge was washed with water (10ml), dried with nitrogen and 3-[¹⁸F] fluoro-1-tritylsulfanyl-propane was eluted with 4 aliquots of acetonitrile (0.5ml per aliquot).

### Step (b): Preparation of 3-[¹⁸F] fluoro-propane-1-thiol

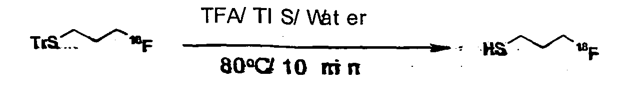

A solution of 3-[ ¹⁸ F] fluoro-1-tritylsulfanyl-propane in acetonitrile (1-2 ml) was evaporated to dryness using a stream of nitrogen at 100°C/10mins. A mixture of TFA (0.05ml), triisopropylsilane (O.Olml) and water (O.Olml) was added followed by heating at 80°C/10mins to produce 3-[¹⁸F] fluoro-propane-1-thiol.

### Step (c): Reaction with -N(CO)CH₂Cl Precursors.

A general procedure for labelling a chloroacetyl precursor is to cool the reaction vessel containing the 3-[¹⁸F] fluoro-1-mercapto-propane from Step (b) with compressed air, and then to add ammonia (27% in water, 0.1ml) and the precursor (1mg) in water (0.05ml). The mixture is heated at 80 °C/ 10mins.

### Example 17: Synthesis of Compounds 45-48.

### Step (a): Synthesis of aminooxy precursors.

Compounds 45 and 47 were synthesised using a manual nitrogen bubbler apparatus on a 0.2 mmol scale starting with Fmoc-protected Rink Amide MBHA resin (Novabiochem). Fmoc amino acids were purchased from Novabiochem and mono disperse Fmoc-PEG amino acids from Polypure AS. Boc-(aminooxy)acetic acid was purchased from Fluka. After attaching Dde-Lys(Fmoc)-OH to the resin the side chain Fmoc group was cleaved followed by coupling of Boc(aminooxy)acetic acid. The Dde group was cleaved by standard hydrazine treatment. Lysine was coupled using HATU/DIEA whereas PyAOP/DIEA was used for all other couplings. Glucose O-acetyl groups were removed by reaction with sodium methoxide in methanol before cleaving the material off the solid support. Simultaneous removal of product from the resin and cleavage of side-chain protecting groups was carried out in TFA containing 2.5% H₂O and 2.5% triisopropylsilane for 1-2 hours. Crude material was purified by preparative HPLC (column Phenomenex Luna C18(2) 5µ 21.2 x 250 mm; solvents A =water/0.1 % TFA and B = acetonitrile/0.1% TFA; suitable gradient over 60 min; flow 10.0 ml/min; UV detection at 214 mn) to give a white solids or viscous, colourless oils after lyophilisation. Identity of the products was confirmed by LC-MS analysis (column Phenomenex Luna C18(2) 3*µ* 2.0 x 50 mm, solvents: A = water/0.1 % TFA and B =acetonitrile/0.1% TFA; suitable gradient over 10 min; flow 0.3 ml/min, UV detection at 214 and 254mn, ESI-MS positive mode)

### Step (b): Conjugation to give non-radioactive fluorine Compounds 46 and 48.

4-Fluorobenzaldehyde and 4-(3-fluoropropoxy)benzaldehyde were purchased from Fluka and Fluorochem, respectively. To a solution of aminooxy precursor (ca 5 µmol) from a) in 20% acetonitrile (3 ml) was added aldehyde (5 fold excess). The mixture was stirred at room temperature for 15 min and concentrated. The product was purified and analysed as under step (a) above.

### Example 18: In Vitro Metalloproteinase inhibition assay.

Compounds were screened using the following commercially available Biomol assay kits:
MMP-1 colorimetric assay kit - Catalogue number AK-404,
MMP-2 colorimetric assay kit - Catalogue number AK-408,
MMP-8 colorimetric assay kit - Catalogue number AK- 414,
MMP-9 colorimetric assay kit - Catalogue number AK-410,
MMP-12 colorimetric assay kit - Catalogue number AK-402,
Which are available from Affiniti Research Products Ltd. (Palatine House, Matford
Court, Exeter, EX2 8NL, UK).

### (a) Test Compound Preparation.

Inhibitors were provided in powdered form, and stored at 4°C. For each inhibitor a 1mM stock solution in DMSO was prepared, dispensed into 20µl aliquots and these aliquots stored at -20°C. The stock solution was diluted to give 8 inhibitor concentrations (recommended: 50µM, 5 µM, 500nM, 50nM, 5nM, 500pM, 50pM and 5pM). Dilutions were made in the kit assay buffer. A five-fold dilution of the inhibitor stocks is made on addition to the assay wells, therefore final concentration range was from 10 µM to 1pM.

### (b) Experimental Procedure.

Details are provided with the commercial kit, but can be summarised as follows:
- Prepare test compound dilutions as above,
- Add assay buffer to plate,
- Add test compounds to plate
- Prepare standard kit inhibitor NNGH (see kit for dilution factor)
- Add NNGH to control inhibitor wells
- Prepare MMP enzyme (see kit for dilution factor)
- Add MMP to plate
- Incubate plate at 37°C for ~15min
- Prepare thiopeptolide substrate (see kit for dilution factor)
- Add substrate to plate
- Count every 2min for 1hr, 37°C, 414nm on a Labsystems iEMS plate reader (for MNM-1 count every 30 seconds for 20 minutes).

### (c) Results.

The results are given in Table 1:

**Table 1**

| **Compound** | **MMP-1 (Ki)** | **MMP-2 (Ki)** | **MMP-8 (Ki)** | **MMP-9 (Ki)** | **MMP-12 (Ki)** |
|---|---|---|---|---|---|
| 20 | - | 0.15 ± 0.06nM (n=2) | - | 0.043 ± 0.02nM (n=2) | 0.11nM |
| 21 | 194.9nM | 1.15 ±. 0.15nM (n=3) | 0.87nM | 0.820± 0.049 (n=3) | - |
| 24 | 4948.5± 2684.9nM (n=2) | 0.14± 0.06nM (n=3) | 3.93nM | 0.58± 0.3nM (n=3) | 0.67± 0.14nM (n=2) |
| 32 | 330nM | 0.62± 0.15nM (n=4) | 2.17nM | 0.37± 0.092nM (n=4) | 00.89± 0.21nM (n=2) |
| 38 | - | 1.25 ± 0.28nM (n=3) | - | 0.99± 0.40nM (n=3) | 0.05nM |
| 42 | - | 2.190± 1.510n M (n=2) | - | 0.585 ± 0.175nM (n=2) | - |
| 44 | - | 0.25 ± 0.12nM (n=3) | - | 0.076± 0.074nM (n=2) | - |
| 46 | 171.7 ± 25:0nM (n=2) | 1.96 ± 1.0nM (n=3) | 0.52nM | 0.44± 0.177nM (n=3) | 0.17nM |
| 48 | 33.3nM | - | 0.50nM | 0.2nM | - |

### Example 19: ^{99m}Tc-radiolabelling (general method).

^{99m}Tc complexes may be prepared by adding the following to a nitrogen-purged P46 vial:
1 ml N₂ purged MeOH,
100µg of the ligand-MMPi conjugate in 100µl MeOH,
0.5ml Na₂CO₃/NaHCO₃ buffer (pH 9.2),
0.5ml TcO₄ from Tc generator,
0.1 ml SnCl₂/MDP solution,
(solution containing 10.2mg SnCl₂ and 101mg methylenediphosphonic acid in 100ml N₂ purged saline).

ITLC (Instant thin layer chromatography) is used to determine the RCP. SG plates and a mobile phase of MeOH/(NH₄OAc 0.1M) 1:1 show RHT (reduced hydrolysed Tc) at the origin, pertechnetate at the solvent front and technetium complexes at an intermediate Rf.

### Example 20: General Procedure for Electrophilic Radioiodination of Precursors.

All precurors were labelled according to the following procedure:

10µL 0.1mM Na¹²⁷I (in 0.01M NaOH, 1 x 10⁻⁹ mol) was added to a vial containing 200 µL 0.2M NH₄OAc buffer (pH 4). This mixture was added to a vial containing Na¹²³I (25.0 µL in 0.05M NaOH, ca. 500 MBq). The combined solution was then transferred to a silanised plastic vial. 5 µL (2.5 x 10⁻⁸mol) of a freshly prepared peracetic acid solution in water (approx. 5mM) was added to the reaction vial. Finally, the precusor (34 µL of a 3mM solution in MeOH) was added to the reaction vial and the solution allowed to stand for 3 min.

The compounds were purified by HPLC.

### HPLC Method:

| | |
|---|---|
| Solvent A: | 0.1% TFA in water |
| Solvent B: | 0.1% TFA in MeCN |
| Column: | Phenomenex Luna 5 µm C18 (2) 150 x 4.6mm. |

### Gradient:

| Time | %B |
|---|---|
| 0.0 | 30 |
| 20.0 | 70 |
| 20.20 | 100 |
| 23.20 | 100 |
| 23.70 | 30 |
| 30.0 | 30 |

**Table 2: HPLC retention times of radioiodinated compounds**

| **Precursor** | **Product name** | **Retention time (min)** |
|---|---|---|
| Compound 23 | Compound 24A | 15.6 |
| Compound 19 | Compound 21A | 7.6 |
| Compound 20 | Compound 20A | 9.4 |
| Compound 31 | Compound 30A | 9.1 |

### Example 21: Synthesis of ¹⁸F-labelled derivatives: Compounds 46B and 48B.

### Step (a): 4-¹⁸F-benzaldehyde.

To a flat-bottom carbon glass reaction vessel (4ml) was added Kryptofix 222 (5mg) in acetonitrile (800ul) and potassium carbonate [13.5mg/ml (H₂O), *ca.* 0.1m] (50µl) were added. The vessel was placed in a brass heater and the reaction vessel lid fitted with 3 PTFE lines was tightened down. Line 1 was fitted with a 2-way tap, line 2 was connected to a waste vial and line 3 was blanked off. The experimental set-up was placed behind a lead wall. ¹⁸F-Fluoride contained in the cyclotron target water (370-740MBq; 0.5-2ml) was added through the two-way tap. The N₂ line was connected to the 2-way tap and the heater was set at 110°C. At 10 min after heating was started, the N₂ line was removed and an aliquot of acetonitrile (0.5ml) was added. This process was repeated at ca. 10.5 and 11 min after heating was started. Following each addition of acetonitrile the N₂ line was reconnected to the 2-way tap. A second nitrogen line was connected to the capped off line 3, to flush out any liquid present in this line. The ¹⁸F-Fluoride was dried up to 30mins in total. After 30mins, the heater was cooled down with compressed air, the reaction vessel lid was removed and 4-(trimethylammonium)benzaldehyde trifluoromethane sulfonate [prepared by the method of Poethko et al, J. Nucl. Med., 45(5) p 892-902 (2004); 0.5-0.8mg; 0.0016-0.0026mmol] in DMSO (1000µl) was added- The 3. PTFE lines were capped off with stoppers. The reaction vessel was heated at 90°C/15min to yield 4-¹⁸F-benzaldehyde (typical incorporation yield ca.50%). The crude product was used without further purification.

### Step (b): Conjugation procedure.

Compound 45 (2mg, 0.003mmol) or Compound 47 (4mg, 0.002mmol) dissolved in citric acid/ Na₂HPO₄ buffer [500µl; prepared by mixing 809 µL of a 0.1M aqueous citric acid solution with 110 µL of a 0.2M aqueous solution of anhydrous Na₂HPO₄], was added directly to 4-¹⁸F-benzaldehyde (crude) from Step (a). The reaction vessel was heated at 70 °C/15mins to yield crude Compound 46B or 48B.

### Step (c): Work-up procedure and Formulation.

The whole reaction mixture form step (b) was diluted with water to a volume of *ca.* 20ml and loaded onto conditioned t-C18 sep pak [conditioned with DMSO(5ml) followed with water(10ml)]. The loaded t-C18 sep was subsequently flushed with water (2x5ml) followed with DMSO (3x5ml). The combined DMSO flushes, containing the desired products, were purified using the RP HPLC preparative system:

| | |
|---|---|
| Column | LunaC18(2) 10×100mm (5u) |
| Eluent | Water (pump A): Acetonitrile (pump B) |
| Loop Size | 2ml |
| Flow rate | 3ml/min |
| Wavelength | 254nm |

Typical retention times for Compound 46B or 48B on the preparative column were 23 and 21mins respectively. The separated HPLC peak was diluted with water to a volume of *ca.* 20ml and loaded onto a conditioned t-C18 sep pak [conditioned with ethanol (5ml) followed with water (10ml)]. The loaded t-C18 sep pak was subsequently flushed with water (1×5ml) followed with ethanol (3×0.2ml, 1×0.4ml). The combined ethanol flush, containing the desired products, was evaporated to a volume *of ca.* 0.1ml and formulated to *ca.* 10% ethanol with phosphate-buffered saline (PBS,1mI). pH of formulated compounds was ca. 7.

### Example 22: Plasma and In Vivo Stability of the ¹²³I Radioiodinated Derivative of Compound 24 (Compound 24A).

Plasma and *in vivo* stability studies were performed with Compound 24A to determine the stability and metabolism of the compound. *In vitro* rat plasma stability demonstrated good stability, with RCP of parent compound changing from 93% to 80% through 2 hours incubation at 37°C.

*In vivo* studies in the rat showed both a slight instability and metabolism of Compound 24A through time *in vivo.* Only plasma and bile samples could be analysed due to insufficient radioactivity within the urine. An increasing amount of free iodide was seen in plasma samples through time, but only a small amount of total activity injected was present. One metabolite was detected in plasma samples and 4 in bile samples, indicating that metabolism was also occurring.

### Example 23: Biodistribution of a Radioiodinated Derivative (Compound 24A) in an LLC Tumour Model In Vivo.

1 x 1.0⁶ Lewis lung carcinoma (LLC) cells are injected subcutaneously into right inner thigh of C57BL/6 mice. Tumours were allowed to grow for 15 days prior to biodistribution being carried out. This model has been shown to expression levels of both active gelatinases (MMP-2) collagenases (MMP-1 and 8) [Bae et al Drugs Exp Clin Res., 29(1):15-23 (2003)].

### Results.

Biodistribution studies were performed in the LLC tumour modeL Compound 24A was initially cleared very rapidly from the blood and was primarily excreted through the hepatobiliary system (HBS). Some retention was seen within tumour tissue, with low background tissue uptake. A summary of the results is given in Table 3 below:

**Table 3: biodistribution of ¹²³ I-labelled Compounds in a LLC tumour model.**

| | **Time Post Injection (minutes)** | | | | | | | |
|---|---|---|---|---|---|---|---|---|
| | **5** | | **30** | | **60** | | **120** | |
| | **Mean** | **STD** | **Mean** | **STD** | **Mean** | **STD** | **Mean** | **STD** |
| **Compound 24A** | | | | | | | | |
| **% ID Urinary** | 7.17 | 0.36 | 6.89 | 1.44 | 5.5 | 2.19 | 7.22 | 0.86 |
| **% ID/g Tumour** | 0.6 | 0.18 | 0.56 | 0.12 | 0.48 | 0.06 | 0.6 | 0.07 |
| **Tumour/Blood** | 0.32 | 0.07 | 0.59 | 0.19 | 0.46 | 0.05 | 0.66 | 0.19 |
| **Tumour/Muscle** | 1.19 | 0.12 | 1.6 | 0.14 | 1.41 | 0.43 | 1.87 | 0.94 |
| **Tumour/Lung** | 0.22 | 0.06 | 0.39 | 0.06 | 0.37 | 0.26 | 0.85 | 0.28 |
| **Tumour/Heart** | 0.27 | 0.04 | 0.57 | 0.12 | 0.68 | 0.07 | 1.23 | 0.26 |

| **Compound 44A** | | | | | | | | |
|---|---|---|---|---|---|---|---|---|
| **% ID Urinary** | 3.94 | 0.04 | 5.85 | 2.22 | 6.36 | 1.16 | 9.42 | 2.37 |
| **% ID/g Tumour** | 1.14 | 0.21 | 0.5 | 0.02 | 0.66 | 0.12 | 0.53 | 0.1 |
| **Tumour/Blood** | 0.41 | 0.15 | 0.38 | 0.13 | 0.42 | 0.12 | 0.43 | 0.06 |
| **Tumour/Muscle** | 1.91 | 1.24 | 1.57 | 0.14 | 2.3 | 0.16 | 2.8 | 0.71 |
| **Tumour/Lung** | 0.49 | 0.16 | 0.54 | 0.11 | 0.69 | 0.16 | 0.72 | 0.16 |
| **Tumour/Heart** | 0.86 | 0.46 | 0.870 | 0.320 | 0.940 | 0.240 | 1.060 | 0.140 |

| **Compound 32A** | | | | | | | | |
|---|---|---|---|---|---|---|---|---|
| **% ID Urinary** | 9.82 | 4.99 | 35.20 | 4.08 | 54.06 | 7.63 | 64.10 | 7.29 |
| **% ID/g Tumour** | 2.58 | 0.27 | 2.67 | 0.43 | 2.03 | 0.37 | 1.34 | 0.25 |
| **Tumour/Blood** | 0.16 | 0.05 | 0.26 | 0.03 | 0.43 | 0.05 | 0.53 | 0.11 |
| **Tumour/Muscle** | 1.66 | 0.43 | 2.80 | 0.68 | 4.88 | 2.10 | 3.92 | 0.60 |
| **Tumour/Lung** | 0.30 | 0.05 | 0.43 | 0.09 | 0.69 | 0.18 | 0.55 | 0.18 |
| **Tumour/Heart** | 0.48 | 0.15 | 0.75 | 0.15 | 1.31 | 0.15 | 1.54 | 0.37 |

| **Compound 38A** | | | | | | | | |
|---|---|---|---|---|---|---|---|---|
| **% ID Urinary** | 6.37 | 0.26 | 40.17 | 8.53 | 68.53 | 5.54 | 78.75 | 1.69 |
| **% ID/g Tumour** | 2.08 | 0.38 | 2.14 | 0.37 | 1.06 | 0.35 | 0.42 | 0.10 |
| **Tumour/Blood** | 0.13 | 0.03 | 0.30 | 0.01 | 0.45 | 0.08 | 0.52 | 0.15 |
| **Tumour/Muscle** | 1.22 | 0.18 | 2.19 | 0.27 | 2.27 | 0.58 | 1.82 | 0.52 |
| **Tumour/Lung** | 0.33 | 0.09 | 0.73 | 0.09 | 0.83 | 0.12 | 0.64 | 0.18 |
| **Tumour/Heart** | 0.29 | 0.02 | 0.90 | 0.12 | 1.25 | 0.52 | 1.14 - | 0.15 |

| **Compound 42A** | | | | | | | | |
|---|---|---|---|---|---|---|---|---|
| **% ID Urinary** | 6.89 | 0.43 | 16.98 | 2.47 | 31.69 | 3.61 | 48.07 | 3.27 |
| **% ID/g Tumour** | 2.26 | 0.46 | 2.67 | 0.37 | 2.35 | 0.40 | 1.29 | 0.24 |
| **Tumour/Blood** | 0.26 | 0.04 | 0.54 | 0.07 | 0.66 | 0.02 | 0.56 | 0.05 |
| **Tumour/Muscle** | 1.46 | 0.22 | 3.22 | 0.09 | 3.52 | 0.54 | 2.51 | 0.87 |
| **Tumour/Lung** | 0.26 | 0.05 | 0.57 | 0.03 | 0.74 | 0.08 | 0.60 | 0.04 |
| **Tumour/Heart** | 0.47 | 0.13 | 1.24 | 0.10 | 1.49 | 0.21 | 1.49 | 0.42 |

| **Compound 21A** | | | | | | | | |
|---|---|---|---|---|---|---|---|---|
| **% ID Urinary** | 5.62 | 1.75 | 26.73 | 3.14 | 39.99 | 3.28 | 52.08 | 3.70 |
| **% ID/g Tumour** | 1.64 | 0.29 | 2.20 | 0.32 | 1.97 | 0.16 | 1.60 | 0.14 |
| **Tumour/Blood** | 0.13 | 0.02 | 0.33 | 0.04 | 0.40 | 0.02 | 0.37 | 0.02 |
| **Tumour/Muscle** | 0.92 | 0.11 | 1.72 | 0.35 | 2.30 | 0.20 | 2-44, | 0.07 |
| **Tumour/Lung** | 0.28 | 0.01 | 0.65 | 0.10 | 0.66 | 0.06 | 0.61 | 0.04 |
| **Tumour/Heart .** | 0.30 | 0.03 | 0.83 | 0.09 | 1.14 | 0.03 | 0.91 | 0.05 |

| | | | | | | | | |
|---|---|---|---|---|---|---|---|---|
| where: STD = standard deviation, ID = injected dose and Urinary = urinary excretion. | | | | | | | | |

### Example 24: Biodistribution of ¹⁸F-labelled Derivatives (Compounds 46B and 48B) in a Tumour Model In: Vivo.

Biodistribution was performed in the LLC tumour model of Example 23 with Compounds 46B and 48B. A summary of the results is given below:

**Table 4: Biodistribution of ¹⁸F-labelled compounds in the LLC model in vivo.**

| | **Time Post Injection (minutes)** | | | | | | | |
|---|---|---|---|---|---|---|---|---|
| | **5** | | **30** | | **60** | | **120** | |
| | **Mean** | **STD** | **Mean** | **STD** | **Mean** | **STD** | **Mean** | **STD** |
| **Compound 46B** | | | | | | | | |
| **% ID Urinary** | 6.60 | 4.24 | 12.00 | 4.17 | 15.02 | 3.11 | 19.68 | 4.54 |
| **% ID/g Tumour** | 0.42 | 0.17 | 0.53 | 0.21 | 0.32 | 0.26 | 0.31 | 0.21 |
| **TumourBlood** | 0.18 | 0.05 | 0.47 | 0.24 | 0.73 | 0.54 | 0.73 | 0.51 |
| **Tumour/Muscle** | 1.16 | 0.12 | 2.06 | 0.63 | 2.02 | 1.10 | 1.81 | 1.00 |
| **Tumour/Lung** | 0.21 | 0.09 | 0.55 | 0.21 | 0.60 | 0.53 | 0.80 | 0.79 |
| **Tumour/Heart** | 0.37 | 0.11 | 1.15 | 0.33 | 1.54 | 1.12 | 1.33 | 1.02 |

| **Compound 48B** | | | | | | | | |
|---|---|---|---|---|---|---|---|---|
| **% ID Urinary** | 0.24 | 0.08 | 15.29 | 6.90 | 56.89 | 14.89 | 59.33 | 10.10 |
| **% ID/g Tumour** | 2.53 | 0.52 | 2.33 | 0.08 | 1.81 | 0.63 | 1.62 | 0.18 |
| **Tumour/Blood** | 0.11 | 1.45 | 0.24 | 0.02 | 0.51 | 0.33 | 0.41 | 0.49 |
| **Tumour/Muscle** | 1.55 | 1.45 | 2.92 | 0.79 | 4.40 | 5.59 | 3.55 | 1.25 |
| **Tumour/Lung** | 0.26 | 0.18 | 0.47 | 0.10 | 0.95 | 0.93 | 0.77 | 0.44 |
| **Tumour/Heart** | 0.40 | 0.40 | 0.84 | 0.09 | 1.69 | 1.20 | 1.52 | 0.83 |

| | | | | | | | | |
|---|---|---|---|---|---|---|---|---|
| where: STD = standard deviation, ID = injected dose and Urinary = urinary excretion. | | | | | | | | |

### Example 25: Biodistribution of ¹²³I- and ¹⁸F-labelled Compounds in a Model of Atherosclerosis In Vivo.

### ApoE Ligation Model.

ApoE -/- mice are transgenic knock-out mice, which lack the ApoE gene, and are therefore unable to regulate their plasma cholesterol levels. As a consequence ApoE mice develop atherosclerotic lesions, a process which is accelerated with feeding of high fat diet. Further acceleration of lesion development can be achieved by ligating the carotid artery, resulting in advanced lesion formation within 4 weeks of surgery and high fat diet feeding. This model has been shown to have have levels of tissue remodelling, with high macrophage and MMP expression, and is described by Ivan et al [Circulation, 105, 2686-2691 (2002)].

Two controls have been used for these experiments: (1) ApoE sham animals in which the mice undergo the same surgical intervention but the sture is only passed beneath the carotid and then removed and , (2) C57BL/6 ligtaed animals which undergo the same surgical ligation and high fat feeding as the ApoE ligated mice. Literature reports have stated that these animals do have some level of tissue remodelling, but with lower active MMP levels [Ivan et al, Circulation, 105, 2686-2691 (2002)].

The results are given in Table 4:

**Table 4: biodistribution of ¹²³I- and ¹⁸F-labelled Compounds in the ApoE model.**

| | **ApoE Ligated** | | | | | **ApoE Sham** | |
|---|---|---|---|---|---|---|---|
| **Time Post Injection** | | | | | | | |
| | **5** | | **60** | | | **60** | |
| | **Mean** | **STD** | **Mean** | **STD** | | **Mean** | **STD** |
| **Compound 24A** | | | | | | | |
| **%ID/G** | | | | | | | |
| **Carotid** | 2.67 | 1.11 | 2.00 | 0.83 | | 0.25 | 0.28 |
| **Carotid/Blood** | 0.94 | 0.22 | 1.97 | 0.99 | | 0.19 | 0.21 |
| **Carotid/Lung** | 0.62 | 0.16 | 1.71 | 0.77 | | 0.17 | 0.18 |
| **Carotid/Heart** | 0.48 | 0.12 | 1.03 | 0.72 | | 0.21 | 0.2 |
| | | | | | | | |

| **Compound 32A** | | | | | | | |
|---|---|---|---|---|---|---|---|
| **%ID/G** | | | | | | | |
| **Carotid** | 10.32 | 2.51 | 10.85 | 2.21 | | 2.91 | 1.08 |
| **Carotid/Blood** | 0.46 | 0.18 | 1.21 | 0.28 | | 0.69 | 0.59 |
| **Carotid/Lung** | 1.01 | 0.47 | 1.45 | 0.70 | | 0.70 | 0.04 |
| **Carotid/Heart** | 1.39 | 0.67 | 3.94 | 0.92 | | 1.28 | 0.49 |
| | | | | | | | |

| **Compound 46B** | | | | | | | |
|---|---|---|---|---|---|---|---|
| **%ID/G** | | | | | | | |
| **Carotid** | 0.88 | 0.31 | 0.44 | 0.13 | | - | - |
| **Carotid/Blood** | 0.42 | 0.16 | 0.61 | 0.40 | | - | - |
| **Carotid/Lung** | 0.35 | 0.00 | 0.53 | 0.44 | | - | - |
| **Carotid/Heart** | 0.49 | 0.04 | 1.11 | 0.83 | | - | - |
| | | | | | | | |

| **Comproud 48B** | | | | | | | |
|---|---|---|---|---|---|---|---|
| **%ID/G** | | | | | | | |
| **Carotid** | 12.01 | 4.33 | 10.34 | 1.19 | | 2.37 | 0.51 |
| **Carotid/Blood** | 0.40 | 0.08 | 0.96 | 0.11 | | 0.2 | 0.09 |
| **Carotid/Lung** | 0.75 | 0.07 | 1.42 | 0.49 | | 0.25 | 0.05 |
| **Carotid/Heart** | 1.27 | 029 | 2.71 | 0.24 | | 0.81 | 0.19 |

| | | | | | | | |
|---|---|---|---|---|---|---|---|
| where: STD = standard deviation and ID = injected dose. | | | | | | | |

### Example 26: Autoradiography of Compounds 24A and 32A in a Model of Atherosclerosis In Vivo.

### Rabbit Cholesterol Model.

New Zealand White rabbits are fed a 1% cholesterol diet for 8 weeks to induce the development of atherosclerosis lesion in the aorta. Validation of this model has shown the development of macrophage rich, advanced atherosclerotic lesions from the aortic arch to the descending aorta. Briefly, Compounds 24A and 32A were injected i.v. into cholesterol fed rabbits, which were euthanized 2 hours p.i. The aorta was removed in toto, and fixed in 10% neutral buffered formalin. Aorta's were opened longitudinally along the ventral midline and stained en face with sudan IV, which detects the presence of atherosclerosis lesions via fat staining. Aorta's were then placed against a phosphor screen overnight. The screen was then scanned the following day to determine areas of radioactivity within the aortic tissue.

The results showed uptake of both compounds into atherosclerotic lesions in the aorta with minimal uptake into normal aortic areas.

### Example 27: Imaging in a Tumour Model In Vivo.

Imaging was performed with Compound 24A in the MDA-MB-231 tumour model (a human breast carcinoma xenograft model). Literature evidence has demonstrated that MDA-MB-231 cells express a range of MMPs, including MMP-1 (pro and active) (Benbow *et al.,* Bacheimer *et al.*), MMP-2 (Bacheimer *et al*.; Lee *et al*), MMP-3 (Bacheimer *et al*.), MMP-7 pro (Bacheimer *et al*.), MMP-9 pro (not active) (Benbow *et al*.; Bacheimer *et al*.; Lee *et al*.; Weber *et al.*), MMP-10, 11 and 14 (all pro) (Benbow *et al*.; Bacheimer *et al*).
Bachmeier et al Anticancer Res. 2001 Nov-Dec;21(6A):3821-8;
Bae et al Drugs Exp Clin Res. 2003;29(1):15-23;
Benbow et al Clin Exp Metastasis.1999 May;17(3):231-8;
Lee et al, Eur. J Cancer, 2001; 37:106-113.
Weber et al Int J Oncol.. 2002 Feb;20(2):299-303.
Tumour "hotspots" were seen from 5 to 120 minutes post injection, with region of interest ratios to muscle greater than 2:1 at all time points. The results are shown in Figure 3.

## Claims

1. An imaging agent which comprises a metalloproteinase inhibitor of Formula (I) labelled with an imaging moiety, wherein the imaging moiety can be detected following administration of said labelled matrix metalloproteinase inhibitor to the mammalian body *in vivo:* 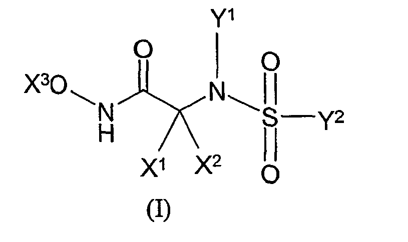 where:
Y¹ is H or -(CH₂)_{w}-(C=_{O})-Z; where w is an integer of value 1 to 6; and Z is OH, C₁₋₆ alkoxy, C₄₋₁₀ aryloxy or NR¹R² wherein R¹ and R² are each independently selected from the group consisting of H, C₁₋₆ alkyl, C₃₋₆ cycloalkyl, C₁₋₆ fluoroalkyl or C₄₋₁₀ aryl.
X¹ and X² together with the carbon atom to which they are attached, form a C₃₋₁₀ saturated ring which may be alicyclic or bicyclic, and may optionally incorporate 1 or 2 heteroatoms chosen from O, N and S;
X³ is H, C₁₋₃ alkyl or C₁₋₃ fluoroalkyl;
Y² is a group of formula -[A¹]ₚ[O]_{q}A² where p and q are 0 or 1, and A¹ is C₁-10 alkylene, C₃₋₈ cycloalkylene, C₁₋₁₀ perfluoroalkylene, C₆₋₁₀ arylene or C₂₋₁₀ heteroarylene, and A² is H, C₁₋₁₀ alkyl, C₃₋₈ cycloalkyl,C₁₋₁₀ perfluoroalkyl, C₆₋₁₀ aryl or C₂₋₁₀ heteroaryl with the proviso that when p=0, q is also 0 and. A² is not H.

2. The imaging agent of Claim 1, where Y¹ is -(CH₂)_{w} (C=O)-Z and w is 1, 2 or 3.

3. The imaging agent of Claims I or 2, where X³ is H, CH₃ or CH₂F.

4. The imaging agent of claims 1 to 3, wherein Y² is -C₆H₄-O-A², and A² is C₆₋₁₀ aryl.

5. The imaging agent of Claims 1 to 4, where the imaging moiety is chosen from:
(i) a radioactive metal ion;
(ii) a paramagnetic metal ion;
(iii) a gamma-emitting radioactive halogen;
(iv) a positron-emitting radioactive non-metal;
(v) a hyperpolarised NMR-active nucleus;
(vi) a reporter suitable *for in vivo* optical imaging;
(vii) a β-emitter suitable for intravascular detection.

6. The imaging agent of Claims 1 to 5, where the imaging agent is of Formula II: 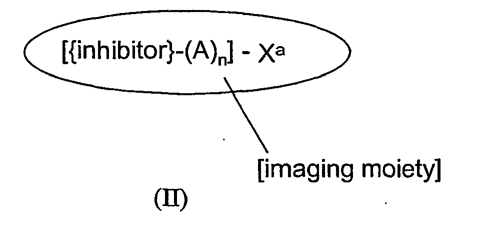 where:
{inhibitor} is the metalloproteinase inhibitor of Formula (I);
-(A)ₙ is a linker group wherein each A is independently -CR₂- , - CR=CR-, -C≡C-, -CR₂CO₂- , -CO₂CR₂- , -NRCO-, -CONR-, - NR(C=O)NR-, -NR(C=S)NR-, -SO₂NR- , -NRSO₂- , -CR₂OCR₂- , -CR₂SCR₂- , -CR₂NRCR₂- , a C₄_₈ cycloheteroalkylene group, a C₄₋₈ cycloalkylene group, a C₅₋₁₂ arylerie group, or a C₃₋₁₂ heteroarylene group, an amino acid, a sugar or a monodisperse polyethyleneglycol (PEG) building block;
R is independently chosen from H,C₁₋₄ alkyl, C₂₋₄ alkenyl, C₂₋₄ alkynyl, C₁₋₄ alkoxyalkyl or C₁₋₄ hydroxyalkyl;
n is an integer of value 0 to 10; and
and X^{a} is H, OH, Hal, NH₂, C₁₋₄ alkyl, C₁₋₄ alkoxy, C₁₋₄ alkoxyalkyl, C₁₋₄ hydroxyalkyl or X^{a} is the imaging moiety.

7. The imaging agent of Claim 6, where the imaging moiety is attached at the Y¹ or Y² positions of the metalloproteinase inhibitor.

8. The imaging agent of Claims 1 to 7, where the matrix metalloproteinase inhibitor is conjugated to a ligand, and said ligand forms a metal complex with the radioactive metal ion or paramagnetic metal ion.

9. The imaging agent of Claim 8, where the ligand is a chelating agent.

10. The imaging agent of Claims 8 or 9, where the radioactive metal ion is a gamma emitter or a positron emitter.

11. The imaging agent of Claim 10, where the radioactive metal ion is ^{99m}Tc, ¹¹¹In, ⁶⁴Cu, ⁶⁷Cu, ⁶⁷Ga or ⁶⁸Ga.

12. The imaging agent of Claim 10, where the gamma-emitting radioactive halogen imaging moiety is ¹²³_{I.}

13. The imaging agent of Claim 10, where the positron-emitting radioactive non-metal is chosen from ¹⁸F, ¹¹C or ¹³N.

14. The imaging agent of Claims 1 to 13, where the matrix metalloproteinase inhibitor is of Formula IV: 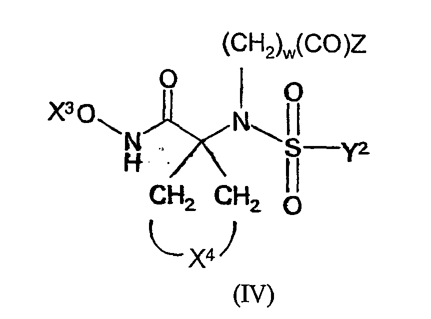 where:
Y², wand Z are as defined in Claim 1;
X³ is H, CH₃ or CH₂F;
X⁴ is -(CH₂)ₘ- where m is 1, 2 or 3, -CH₂OCH₂- or X⁵ where X⁵ is 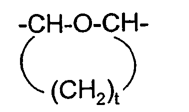
where t is 2 or 3.

15. The imaging agent of Claim 14, where Z is NR¹R².

16. The imaging agent of Claims 14 or 15, where the matrix metalloproteinase inhibitor is of Formula V: 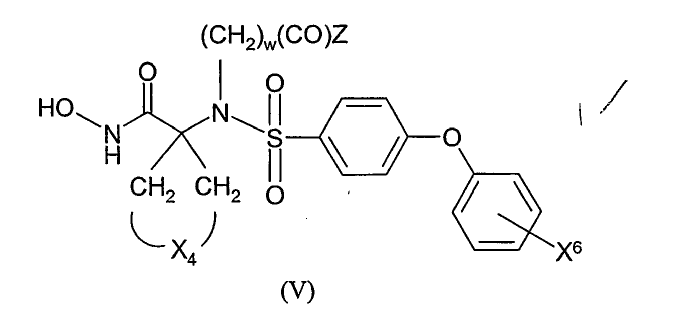 where:
X⁶ is Hal, R¹ or OR¹, where R¹ is C₁₋₃ alkyl or C₁₋₃ fluoroalkyl.

17. The imaging agent of Claim 16, where Z is NR¹R², X⁶ is F; and X⁴ is -(CH₂)₂-, -CH₂OCH₂- or X⁵ with t equal to 2.

18. A pharmaceutical composition which comprises the imaging agent of claims 1 to 17 together with a biocompatible carrier, in a form suitable for mammalian administration.

19. A radiopharmaceutical composition which comprises the imaging agent of claims 1 to 17 wherein the imaging moiety is radioactive, together with a biocompatible carrier, in a form suitable for mammalian administration.

20. The radiopharmaceutical composition of claim 19, where the imaging moiety comprises a radioactive metal ion.

21. The radiopharmaceutical composition of claim 19, where the imaging moiety comprises a positron-emitting radioactive non-metal or a gamma-emitting radioactive halogen.

22. A conjugate of a matrix metalloproteinase inhibitor of Formula (I) as defined in Claim 1 with a ligand, wherein said ligand is capable of forming a metal complex with a radioactive or paramagnetic metal ion.

23. The conjugate of Claim 20, of Formula IIb: 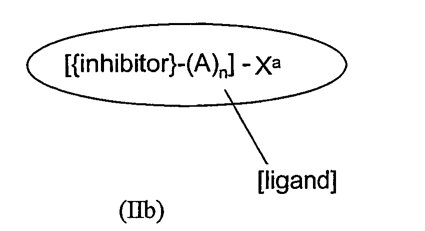 where {inhibitor}. A, n and X^{a} are as defined in Claim 6.

24. The conjugate of Claims 22 or 23, wherein the matrix metalloproteinase inhibitor is of Formulae IV or V of Claims 14 to 17.

25. The conjugate of Claims 22 to 24, wherein the ligand is a chelating agent.

26. The conjugate of Claim 25, wherein the chelating agent has a diaminedioxime, N₂S₂, or N₃S donor set.

27. A kit for the preparation of the radiopharmaceutical composition of Claim 20, which comprises the conjugate of Claims 22 to 26.

28. The kit of Claim 30, where the radioactive metal ion is ^{99m}Tc, and the kit further comprises a biocompatible reductant.

29. A kit for the preparation of the radiopharmaceutical composition of Claim 21, which comprises a precursor, said precursor being a non-radioactive derivative of the matrix metalloproteinase inhibitor of claims 1 to 17, wherein said non-radioactive derivative is capable of reaction with a source of the positron-emitting radioactive non-metal or gamma-emitting radioactive halogen to give the desired radiopharmaceutical.

30. The kit of claim 29 where the precursor is in sterile, apyrogenic form.

31. The kit of Claims 29 or 30, where the source of the positron-emitting radioactive non-metal or gamma-emitting radioactive halogen is chosen from:
(i) halide ion or F⁺ or I⁺; or
(ii) an alkylating agent chosen from an alkyl or fluoroalkyl halide, tosylate, triflate or mesylate.

32. The kit of Claims 29 to 31, where the non-radioactive derivative is chosen from:
(i) an organometallic derivative such as a trialkylstannane or a trialkylsilane;
(ii) a derivative containing an alkyl halide, alkyl tosylate or alkyl mesylate for nucleophilic substitution;
(iii) a derivative containing an aromatic ring activated towards nucleophilic or electrophilic substitution;
(iv) a derivative containing a functional group which undergoes facile alkylation;
(v) a derivative which alkylates thiol-containing compounds to give a thioether-containing product.

33. The kit of claims 29 to 32, where the precursor is bound to a solid phase.

34. Use of the imaging agent of Claims 1 to 17 for the diagnostic imaging of atherosclerosis.

35. Use of the imaging agent of Claims 1 to 17 for the diagnostic imaging of unstable plaques.

36. Use of the imaging agent of Claims 1 to 17 for the intravascular detection of atherosclerosis.

## Patentansprüche

1. Bildgebungsmittel, umfassend einen Metalloproteinase-Inhibitor der Formel (1), markiert mit einer Bildgebungseinheit, wobei die Bildgebungseinheit detektiert werden kann nach der Verabreichung des markierten Matrix-Metalloproteinase-Inhibitors an einen Körper eines Säugers in vivo: 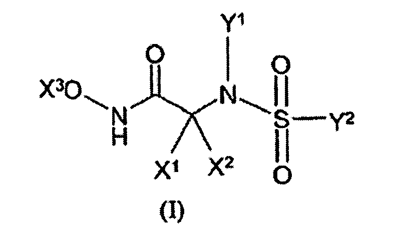 wobei:
Y¹ steht für H oder -(CH₂)_{w}-(C=O)-Z; wobei w eine ganze Zahl des Wertes 1 bis 6 ist; und
Z steht für OH, C₁₋₆-Alkoxy, C₄₋₁₀-Aryloxy oder NR¹R², wobei R¹ und R² jeweils unabhängig ausgewählt sind aus der Gruppe, die besteht aus H, C₁₋₆-Alkyl, C₃₋₆-Cycloalkyl, C₁₋₆-Fluoroalkyl oder C₄₋₁₀-Aryl.
X¹ und X² zusammen mit dem Kohlenstoffatom, an das sie gebunden sind, einen gesättigten C₃₋₁₀-Ring bilden, der alicyclisch oder bicyclisch sein kann, und ggf. ein oder zwei Heteroatome eingebaut haben, ausgewählt aus O, N und S;
X³ steht für H, C₁₋₃-Alkyl oder C₁₋₃-Fluoroalkyl;
Y² steht für eine Gruppe der Formel -[A¹]ₚ[O]_{q}A², wobei p und q für 0 oder 1 stehen, und A¹ steht für C₁₋₁₀-Alkylen, C₃₋₈-Cycloalkylen, C₁₋₁₀-Perfluoralkylen, C₆₋₁₀-Arylen oder C₂₋₁₀-Heteroarylen, und A² steht für H, C₁₋₁₀-Alkyl, C₃₋₈-Cycloalkyl, C₁₋₁₀-Perfluoralkyl, C₆₋₁₀-Aryl oder C₂₋₁₀-Heteroaryl, mit der Maßgabe, dass wenn p=0, q auch für 0 steht und A² nicht für H steht.

2. Bildgebungsmittel nach Anspruch 1, wobei Y¹ steht für -(CH₂)_{w}-(C=O)-Z und w steht für 1, 2 oder 3.

3. Bildgebungsmittel nach Anspruch 1 oder 2, wobei X¹ steht für H, CH₃ oder CH₂F.

4. Bildgebungsmittel nach den Ansprüchen 1 bis 3, wobei Y² steht für -C₆H₄-O-A², und A² steht für C₆₋₁₀-Aryl.

5. Bildgebungsmittel der Ansprüche 1 bis 4, wobei die Bildgebungseinheit ausgewählt ist aus:
(i) einem radioaktiven Metallion;
(ii) einem paramagnetischen Metallion;
(iii) einem Gamma-emittierenden radioaktiven Halogen,
(iv) einem Positron-emittierenden radioaktiven Nicht-Metall;
(v) einem hyperpolarisierten NMR-aktiven Kern;
(vi) einem Reporter, der für eine optische in-vivo-Bildgebung geeignet ist;
(vii) einem β-Emitter, der für intravaskuläre Detektion geeignet ist.

6. Bildgebungsmittel nach Anspruch 1 bis 5, wobei das Bildgebungsmittel von der Formel II ist: 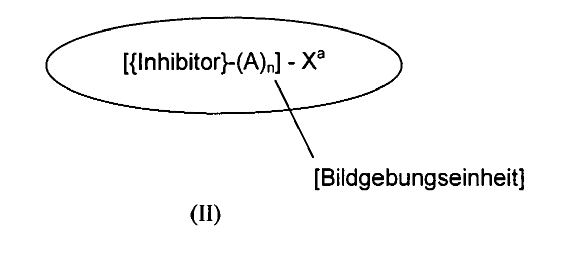 wobei:
{Inhibitor} der Metalloproteinase-Inhibitor der Formel (I) ist;
-(A)ₙ- eine Linkergruppe ist, wobei jedes A unabhängig steht für -CR₂- , -CR=CR- , -C≡C- , -CR₂CO₂- , -CO₂CR₂- , -NRCO- , -CONR- , NR(C=O)NR- , -NR(C=S)NR- , -SO₂NR- , -NRSO₂- , -CR₂OCR₂- , -CR₂SCR₂- , -CR₂NRCR₂- , eine C₄₋₈-Cycloheteroalkylen-Gruppe, eine C₄₋₈-Cycloalkylen-Gruppe, eine C₅₋₁₂-Arylen-Gruppe, oder eine C₃₋₁₂-Heteroarylen-Gruppe, eine Aminosäure, einen Zucker oder einen monodispersen Polyethylenglycol(PEG)-Bildungsblock;
R unabhängig ausgewählt ist aus H, C₁₋₄-Alkyl, C₂₋₄-Alkenyl, C₂₋₄-Alkinyl, C₁₋₄-Alkoxylalkyl oder C₁₋₄-Hydroxyalkyl ;
n eine ganze Zahl des Wertes 0 bis 10 ist; und
X³ steht für H, OH, Hal, NH₂, C₁₋₄-Alkyl, C₁₋₄-Alkoxy, C₁₋₄-Alkoxyalkyl, C₁₋₄-Hydroxyalkyl oder X³ die Bildgebungseinheit ist.

7. Bildgebungsmittel nach Anspruch 6, wobei die Bildgebungseinheit gebunden ist an die Y¹-oder Y²-Positionen des Metalloproteinase-Inhibitors.

8. Bildgebungsmittel nach Anspruch 1 bis 7, wobei der Matrix-Metalloproteinase-Inhibitor konjugiert ist an einen Liganden, und der Ligand einen Metallkomplex mit dem radioaktiven Metallion oder paramagnetischen Metallion bildet.

9. Bildgebungsmittel nach Anspruch 8, wobei der Ligand ein Chelatbildner ist.

10. Bildgebungsmittel nach Anspruch 8 oder 9, wobei das radioaktive Metallion ein Gamma-Emitter oder ein Positron-Emitter ist.

11. Bildgebungsmittel nach Anspruch 10, wobei das radioaktive Metallion steht für ^{99m}Tc, ¹¹¹In, ⁶⁴Cu, ⁶⁷Cu, ⁶⁷Ga oder ⁶⁸Ga.

12. Bildgebungsmittel nach Anspruch 10, wobei die Gamma-emittierende radioaktive Halogen-Bildgebungseinheit für ¹²³I steht.

13. Bildgebungsmittel nach Anspruch 10, wobei das Positron-emittierende radioaktive Nicht-Metall ausgewählt ist aus ¹⁸ F, ¹¹C oder ¹³ N.

14. Bildgebungsmittel nach den Ansprüchen 1 bis 13, wobei der Matrix-Metalloproteinase-Inhibitor von der Formel IV ist: 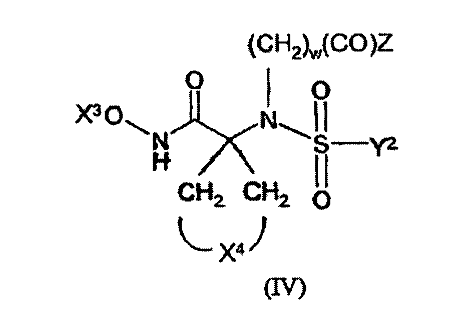 wobei:
Y², w und Z sind wie definiert in Anspruch 1;
X³ steht für H, CH₃ oder CH₂F;
X⁴ steht für -(CH₂)ₘ-, wobei m steht für 1, 2 oder 3, -CH₂OCH₂- oder X⁵, wobei X⁵ steht für 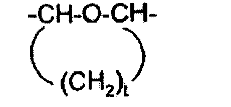
wobei t für 2 oder 3 steht.

15. Bildgebungsmittel nach Anspruch 14, wobei Z für NR¹R² steht.

16. Bildgebungsmittel nach Anspruch 14 oder 15, wobei der Matrix-Metalloproteinase-Inhibitor von der Formel V ist: 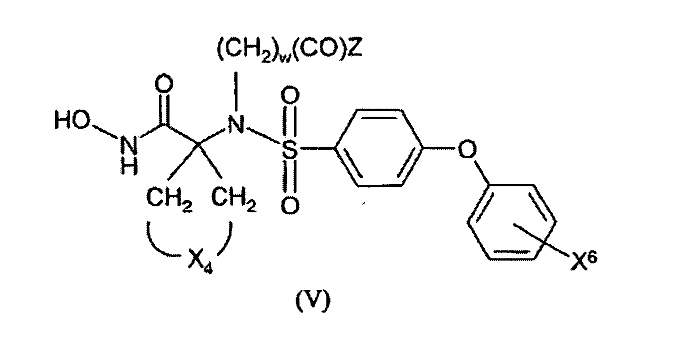 wobei:
X⁶ für Hal steht, R¹ oder OR¹, wobei R¹ für C₁₋₃-Alkyl oder C₁₋₃-Fluoroalkyl steht.

17. Bildgebungsmittel nach Anspruch 16, wobei Z für NR¹R² steht, X⁶ für F steht; und X⁴ für-(CH₂)₂-, -CH₂OCH₂- oder X⁵ steht, mit t gleich 2.

18. Pharmazeutische Zusammensetzung, umfassend das Bildgebungsmittel der Ansprüche 1 bis 17 zusammen mit einem biokompatiblen Träger, in einer Form, die für die Verabreichung an einen Säuger geeignet ist.

19. Radiopharmazeutische Zusammensetzung, umfassend das Bildgebungsmittel der Ansprüche 1 bis 17, wobei die Bildgebungseinheit radioaktiv ist, zusammen mit einem biokompatiblen Träger, in einer Form, die für die Verabreichung an einen Säuger geeignet ist.

20. Radiopharmazeutische Zusammensetzung nach Anspruch 19, wobei die Bildgebungseinheit ein radioaktives Metallion umfasst.

21. Radiopharmazeutische Zusammensetzung nach Anspruch 19, wobei die Bildgebungseinheit ein Positron-emittierendes radioaktives Nicht-Metall oder ein Gamma-emittierendes radioaktives Halogen umfasst.

22. Kunjugat eines Matrix-Metalloproteinase-Inhibitors der Formel (I), wie definiert in Anspruch 1, mit einem Liganden, wobei der Ligand fähig ist, einen Metallkomplex mit einem radioaktiven oder paramagnetischen Metallion zu bilden.

23. Konjugat nach Anspruch 20 der Formel IIb: 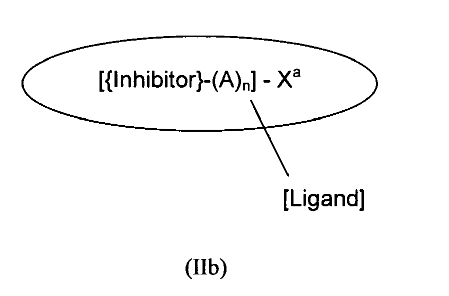 wobei {Inhibitor}, A, n und X^{a} sind wie definiert in Anspruch 6.

24. Konjugat nach Anspruch 22 oder 23, wobei der Matrix-Metalloproteinase-Inhibitor von der Formel IV oder V der Ansprüche 14 bis 17 ist.

25. Konjugat nach den Ansprüchen 22 bis 24, wobei der Ligand ein Chelatbildner ist.

26. Konjugat nach Anspruch 25, wobei der Chelatbildner einen Diamindioxim-, N₂S₂,- oder N₃S-Donorsatz aufweist.

27. Kit für die Herstellung der radiopharmazeutischen Zusammensetzung nach Anspruch 20, umfassend das Kunjugat der Ansprüche 22 bis 26.

28. Kit nach Anspruch 30, wobei das radioaktive Metallion steht für ^{99m}Tc, und das Kit weiter umfasst ein biokompatibles Reduktionsmittel.

29. Kit zur Herstellung der radiopharmazeutischen Zusammensetzung nach Anspruch 21, umfassend einen Vorläufer, wobei der Vorläufer ein nichtradioaktives Derivat des Matrix-Metalloproteinase-Inhibitors der Ansprüche 1 bis 17 ist, wobei das nicht-radioaktive Derivat fähig ist zur Reaktion mit einer Quelle des Positron-emittierenden radioaktiven Nicht-Metalles oder Gamma-emittierenden radioaktiven Halogens, um das erwünschte Radiopharmazeutikum zu ergeben.

30. Kit nach Anspruch 29, wobei der Vorläufer in steriler, apyrogener Form vorliegt.

31. Kit nach Anspruch 29 oder 30, wobei die Quelle des Positron-emittierenden radioaktiven Nicht-Metalles oder Gamma-emittierenden radioaktiven Halogens ausgewählt ist aus:
(i) Halogenidion oder F⁺ oder I⁺; oder
(ii) ein Alkylierungsmittel, ausgewählt aus einem Alkyl- oder Fluoroalkyl-Halogenid, -Tosylat, -Triflat oder -Mesylat.

32. Kit nach den Ansprüchen 29 bis 31, wobei das nicht-radioaktive Derivat ausgewählt ist aus:
(i) einem organometallischen Derivat, wie ein Trialkylstannan oder ein Trialkylsilan;
(ii) einem Derivat enthaltend ein Alkylhalogenid, Alkyltosylat oder Alkylmesylat für nukleophile Substitution;
(iii) einem Derivat, enthaltend einen aromatischen Ring, der in Richtung nukleophiler oder elektrophiler Substitution aktiviert ist;
(iv) einem Derivat, enthaltend eine funktionelle Gruppe, die eine leichte Alkylierung durchläuft;
(v) einem Derivat, das Thiol-enthaltende Verbindungen alkyliert, um ein Thioether-enthaltendes Produkt zu ergeben.

33. Kit nach den Ansprüchen 29 bis 32, wobei der Vorläufer an eine feste Phase gebunden ist.

34. Verwendung des Bildgebungsmittels der Ansprüche 1 bis 17 für die diagnostische Bildgebung von Atherosklerose.

35. Verwendung des Bildgebungsmittels der Ansprüche 1 bis 17 für die diagnostische Bildgebung instabiler Plaques.

36. Verwendung des Bildgebungsmittels der Ansprüche 1 bis 17 für die intravaskuläre Detektion von Atherosklerose.

## Revendications

1. Agent d'imagerie comprenant un inhibiteur de la métalloprotéinase répondant à la formule (I) marqué par un fragment d'imagerie, le fragment d'imagerie étant susceptible d'être détecté à la suite de l'administration dans le corps d'un mammifère *in vivo* dudit inhibiteur de la métalloprotéinase matricielle marqué : 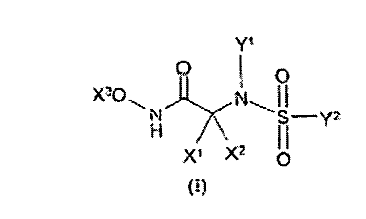 où :
Y¹ représente H ou -(CH₂)_{w}-(C=O)-Z ; w étant un nombre entier d'une valeur allant de 1 à 6 ; et
Z représente OH, alcoxy en C₁-C₆, aryloxy en C₄-C₁₀ ou NR¹R² où R¹ et R² sont chacun indépendamment choisis dans le groupe constitué par H, alkyle en C₁-C₆, cycloalkyle en C₃-C₆, fluoroalkyle en C₁-C₆ ou aryle en C₄-C₁₀.
X¹ et X² forment, conjointement à l'atome de carbone auquel ils sont rattachés, un cycle saturé en C₃-C₁₀ qui peut être alicyclique ou bicyclique, et peut éventuellement comporter 1 ou 2 hétéroatomes choisis parmi O, N et S ;
X³ représente H, alkyle en C₁-C₃ ou fluoroalkyle en C₁-C₃;
Y² représente un groupe répondant à la formule -[A¹]ₚ[O]_{q}A² dans laquelle p et q valent 0 ou 1, et A¹ représente alkylène en C₁-C₁₀, cycloalkylène en C₃-C₈, perfluoroalkylène en C₁-C₁₀, arylène en C₆-C₁₀ ou hétéroarylène en C₂-C₁₀, et A² représente H, alkyle en C₁-C₁₀, cycloalkyle en C₃-C₈, perfluoroalkyle en C₁-C₁₀, aryle en C₆-C₁₀ ou hétéroaryle en C₂-C₁₀, à la condition que lorsque p = 0, q vaut également 0 et A² ne représente pas H.

2. Agent d'imagerie selon la revendication 1, dans lequel Y¹ représente -(CH₂)_{w}-(C=O)-Z et w vaut 1, 2 ou 3.

3. Agent d'imagerie selon les revendications 1 ou 2, dans lequel X³ représente H, CH₃ ou CH₂F.

4. Agent d'imagerie selon les revendications 1 à 3, dans lequel Y² représente - C₆H₄-O-A², et A² représente aryle en C₆-C₁₀.

5. Agent d'imagerie selon les revendications 1 à 4, dans lequel le fragment d'imagerie est choisi parmi :
(i) un ion métallique radioactif ;
(ii) un ion métallique paramagnétique ;
(iii) un halogène radioactif à émission gamma ;
(iv) un non métal radioactif à émission de positrons ;
(v) un noyau actif en RMN hyperpolarisé ;
(vi) un rapporteur approprié à l'imagerie optique *in vivo ;*
(vii) un émetteur β approprié à la détection intravasculaire.

6. Agent d'imagerie selon les revendications 1 à 5, dans lequel l'agent d'imagerie répond à la formule II : 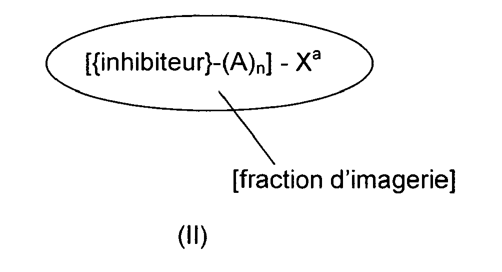 dans laquelle :
{inhibiteur} représente l'inhibiteur de la métalloprotéinase répondant à la formule (I) ;
-(A)ₙ- représente un groupe de liaison dans lequel chaque A représente indépendamment -CR₂-, -CR=CR-, -C≡C-, -CR₂CO₂-, -CO₂CR₂-, -NRCO-, - CONR-, -NR(C=O)NR-, -NR(C=S)NR-, -SO₂NR-, -NRSO₂-, -CR₂OCR₂-, - CR₂SCR₂-, -CR₂NRCR₂-, un groupe cyclohétéroalkylène en C₄-C₈, un groupe cycloalkylène en C₄-C₈, un groupe arylène en C₅-C₁₂ ou un groupe hétéroarylène en C₃-C₁₂, un acide aminé, un sucre ou un bloc constitutif polyethylèneglycol (PEG) monodispersé ;
R est choisi indépendamment parmi H, alkyle en C₁-C₄, alcényle en C₂-C₄, alcynyle en C₂-C₄, alcoxyalkyle en C₁-C₄ ou hydroxyalkyle en C₁-C₄ ;
n représente un nombre entier d'une valeur allant de 0 à 10 ; et
X^{a} représente H, OH, Hal, NH₂, alkyle en C₁-C₄, alcoxy en C₁-C₄, alcoxyalkyle en C₁-C₄, hydroxyalkyle en C₁-C₄ ou bien X^{a} représente le fragment d'imagerie.

7. Agent d'imagerie selon la revendication 6, dans lequel le fragment d'imagerie est rattaché aux positions Y¹ ou Y² de l'inhibiteur de la métalloprotéinase.

8. Agent d'imagerie selon les revendications 1 à 7, dans lequel l'inhibiteur de la métalloprotéinase matricielle est conjugué à un ligand, et ledit ligand forme un complexe métallique avec l'ion métallique radioactif ou l'ion métallique paramagnétique.

9. Agent d'imagerie selon la revendication 8, dans lequel le ligand est un agent chélatant.

10. Agent d'imagerie selon les revendications 8 ou 9, dans lequel l'ion métallique radioactif est un émetteur gamma ou un émetteur de positrons.

11. Agent d'imagerie selon la revendication 10, dans lequel l'ion métallique radioactif est ^{99m}Tc, ¹¹¹In, ⁶⁴Cu, ⁶⁷Cu, ⁶⁷Ga ou ⁶⁸Ga.

12. Agent d'imagerie selon la revendication 10, dans lequel le fragment d'imagerie halogène radioactif à émission gamma est ¹²³I.

13. Agent d'imagerie selon la revendication 10, dans lequel le non métal radioactif à émission de positrons est choisi parmi ¹⁸F, ¹¹C ou ¹³ N.

14. Agent d'imagerie selon les revendications 1 à 13, dans lequel l'inhibiteur de la métalloprotéinase matricielle répond à la formule IV : 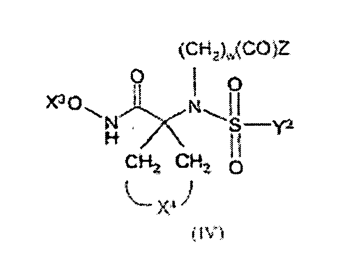 dans laquelle :
Y² , w et Z sont tels que définis dans la revendication 1 ;
X³ représente H, CH₃ ou CH₂F ;
X⁴ représente -(CH₂)ₘ- où m vaut 1, 2 ou 3, -CH₂OCH₂ ou X⁵, X⁵ représentant 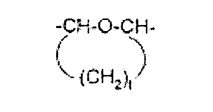
où t vaut 2 ou 3.

15. Agent d'imagerie selon la revendication 14, dans lequel Z représente NR¹R².

16. Agent d'imagerie selon les revendications 14 ou 15, dans lequel l'inhibiteur de la métalloprotéinase matricielle répond à la formule V : 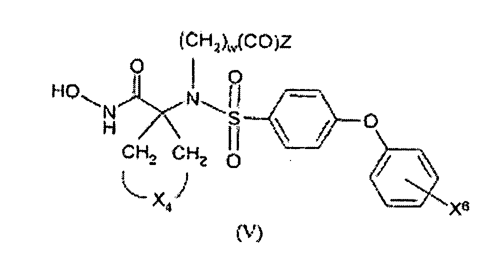 dans laquelle :
X⁶ représente Hal, R¹ ou OR¹, R¹ représentant alkyle en C₁-C₃ ou fluoroalkyle en C₁-C₃.

17. Agent d'imagerie selon la revendication 16, dans lequel Z représente NR¹R², X⁶ représente F ; et X⁴ représente -(CH₂)₂-, -CH₂OCH₂- ou X⁵ dans lequel t vaut 2.

18. Composition pharmaceutique comprenant l'agent d'imagerie selon les revendications 1 à 17 ainsi qu'un véhicule biocompatible, sous une forme appropriée à l'administration à un mammifère.

19. Composition radiopharmaceutique comprenant l'agent d'imagerie selon les revendications 1 à 17, dans laquelle le fragment d'imagerie est radioactif, ainsi qu'un véhicule biocompatible, sous une forme appropriée à l'administration à un mammifère.

20. Composition radiopharmaceutique selon la revendication 19, dans laquelle le fragment d'imagerie comprend un ion métallique radioactif.

21. Composition radiopharmaceutique selon la revendication 19, dans laquelle le fragment d'imagerie comprend un non métal radioactif à émission de positrons ou un halogène radioactif à émission gamma.

22. Conjugué entre un inhibiteur de la métalloprotéinase matricielle répondant à la formule (1), telle que définie dans la revendication 1, et un ligand, ledit ligand étant susceptible de former un complexe métallique avec un ion métallique radioactif ou paramagnétique.

23. Conjugué selon la revendication 20, répondant à la formule IIb : 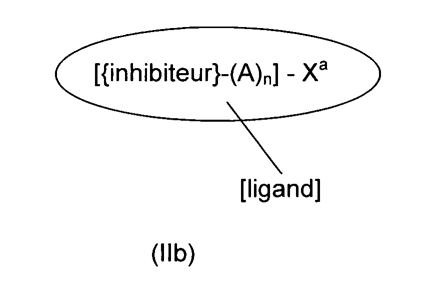 dans laquelle {inhibiteur}, A, n et X^{a} sont tels que définis dans la revendication 6.

24. Conjugué selon les revendications 22 ou 23, dans lequel l'inhibiteur de la métalloprotéinase matricielle répond aux formules IV ou V selon les revendications 14 à 17.

25. Conjugué selon les revendications 22 à 24, dans lequel le ligand est un agent chélatant.

26. Conjugué selon la revendication 25, dans lequel l'agent chélatant possède un ensemble donneur diaminedioxime, N₂S₂, ou N₃S.

27. Trousse pour la préparation de la composition radiopharmaceutique selon la revendication 20, comprenant le conjugué selon les revendications 22 à 26.

28. Trousse selon la revendication 30, dans laquelle l'ion métallique radioactif est ^{99m}Tc, ladite trousse comprenant en outre un réducteur biocompatible.

29. Trousse pour la préparation de la composition radiopharmaceutique selon la revendication 21, comprenant un précurseur, ledit précurseur étant un dérivé non radioactif de l'inhibiteur de la métalloprotéinase matricielle selon les revendications 1 à 17, ledit dérivé non radioactif étant susceptible de réagir avec une source du non métal radioactif à émission de positrons ou de l'halogène radioactif à émission gamma pour donner le produit radiopharmaceutique souhaité.

30. Trousse selon la revendication 29, dans laquelle le précurseur est sous une forme stérile apyrogène.

31. Trousse selon les revendications 29 ou 30, dans laquelle la source du non métal radioactif à émission de positrons ou de l'halogène radioactif à émission gamma est choisie parmi :
(i) un ion halogénure ou F⁺ ou 1⁺ ; ou
(ii) un agent alkylant choisi parmi un tosylate, triflate, mésylate ou halogénure d'alkyle ou de fluoroalkyle.

32. Trousse selon les revendications 29 à 31, dans laquelle le dérivé non radioactif est choisi parmi :
(i) un dérivé organométallique tel qu'un trialkylstannane ou un trialkylsilane ;
(ii) un dérivé contenant un halogénure d'alkyle, tosylate d'alkyle ou mésylate d'alkyle pour une substitution nucléophile ;
(iii) un dérivé contenant un cycle aromatique activé en faveur d'une substitution nucléophile ou électrophile ;
(iv) un dérivé contenant un groupe fonctionnel subissant une alkylation facile ;
(v) un dérivé qui alkyle les composants contenant un thiol pour donner un produit contenant un thioéther.

33. Trousse selon les revendications 29 à 32, dans laquelle le précurseur est lié à une phase solide.

34. Utilisation de l'agent d'imagerie selon les revendications 1 à 17 pour l'imagerie de diagnostic de l'athérosclérose.

35. Utilisation de l'agent d'imagerie selon les revendications 1 à 17 pour l'imagerie de diagnostic des plaques instables.

36. Utilisation de l'agent d'imagerie selon les revendications 1 à 17 pour la détection intravasculaire de l'athérosclérose.
